(19)

**Europäisches Patentamt**

**European Patent Office**

**Office européen des brevets**

(11) **EP 4 710 897 A1**

(12) **EUROPEAN PATENT APPLICATION**

(43) Date of publication:
18.03.2026 Bulletin 2026/12

(21) Application number: 25192881.8

(22) Date of filing: 30.07.2025

(51) International Patent Classification (IPC):
*A61F 2/32* (2006.01)      *A61F 2/36* (2006.01)

(52) Cooperative Patent Classification (CPC):
**A61F 2/3609; A61F 2/32;** A61F 2002/30079;
A61F 2002/3233; A61F 2002/365

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: 13.09.2024  US 202463694572 P
16.01.2025  US 202519026007

(71) Applicant: **Fellowship of Orthopaedic
Researchers, Inc.
Metairie LA 70005 (US)**

(72) Inventors:
• **COOK, Stephen D.
Metairie, 70005 (US)**
• **NOLAN, Liam P.
Metairie, 70005 (US)**
• **SALKELD, Samantha L.
Metairie, 70005 (US)**

(74) Representative: **D Young & Co LLP
3 Noble Street
London EC2V 7BQ (GB)**

(54) **TOTAL HIP REPLACEMENT SYSTEM WITH ROTATABLE FEMORAL MAGNET**

(57)   A hip prosthesis that comprises an acetabular component (1) and a femoral component (21). The acetabular component comprises a shell (2) and optionally a liner (9). The shell has an approximate shape of a hollowed spherical cap, in which the shell comprises one or more magnets (13). The femoral component comprises a stem portion (22), a neck portion (25) that is joined to the stem portion, and a spherical head (28) that is affixed to the neck portion and comprises a spherical magnet (34) that is rotatable within the spherical head. The acetabular component is configured to receive all or a portion of the spherical head of the femoral component. Further, the one or more magnets of the acetabular component and the spherical magnet of the spherical head of the femoral component are oriented to generate an attractive force therebetween.

A

**FIG. 4**

EP 4 710 897 A1

**Description**

## CROSS-REFERENCE TO RELATED APPLICATIONS

This application claims the benefit of U.S. Nonprovisional Application No. 19/026,007, filed on January 16, 2025, and U.S. Provisional Application No. 63/694,572, filed on September 13, 2024, each of which is herein incorporated by reference in its entirety.

## BACKGROUND OF THE INVENTION

The hip joint, also referred to as the acetabulofemoral joint, is between the femur and the acetabulum of the pelvis. The joint is very durable, capable of tolerating high functional loads, large ranges in hip motion, and millions of cycles of repetitive use over a lifetime. As with any joint, it is also capable of repair and recovery from daily use. However, the motion of the hip joint can also become painful and/or limited, due to damage, disease, or injury.

Total hip replacement (THR) is a treatment for a damaged or diseased hip joint, and is considered as one of the most successful surgical procedures in the field of orthopedics, providing significant pain relief, improved quality of life, and increased mobility. The prosthesis used for THR comprises a femoral component and an acetabular component to resemble the ball-and-socket anatomy of the hip joint. The femoral component generally features a stem with a neck that attaches to a ball (*i.e.*, femoral component head), which fits into the acetabular component having the general shape of a cup or shell. Stability of the hip joint is achieved by the ball-and-socket design and the soft tissues and musculature surrounding the hip joint.

A major concern following hip replacement surgery is the dislocation of the femoral component from the acetabular component, which can dramatically affect a patient's quality of life. Dislocation often occurs during motion of the hip joint, *i.e.,* when the femoral component and the acetabular component are not aligned. The prevalence of dislocation has been reported to range from 0.2% to 10% after primary THR (*i.e.*, the first hip replacement surgery) [1, 2], most often occurring in the first six to eight weeks when the soft tissues are healing. But dislocation rate increases substantially to 28% after revision THR (*i.e.*, subsequent hip replacement surgery that removes some or all of the parts of the original prosthesis), with 70% of dislocations occurring within the first month and 75-90% of those occur posteriorly [3]. Risk factors for dislocation include both patient-based factors such as neuromuscular and cognitive disorders, non-compliance with postoperative instructions, advanced age, and the female gender [4]; and surgical-based factors such as the approach, soft tissue tensioning, femoral and acetabular component positioning, impingement, head size, acetabular liner profile, and surgeon experience [5].

Various prosthesis design features have been developed to reduce the incidence of THR dislocation, but these solutions have their own complications and limitations [6]. For example, acetabular liners with a posteriorly oriented rim can provide greater capture of the femoral head within the acetabular shell, but it is accompanied by an increased risk of impingement due to contact occurring between the neck of the femoral component stem and the acetabular component, as well as liner wear, osteolysis, and loosening [7; 8]. Larger femoral component heads have theoretical advantages in regard to stability and reducing dislocation, as the improved head-to-neck ratio can reduce impingement and the larger size indicates that the femoral head can be positioned deeper within the acetabular liner and would require a greater translation "jump distance" before dislocation [2]; however, larger femoral component heads can cause liner wear, which can lead to osteolysis and loosening, and can increase the potential for adverse local tissue reaction secondary to increased corrosion at the junction between the femoral head and neck due to higher torsional forces created by the larger head size. Constrained femoral head-polyethylene liner may help restore stability and reduce dislocation in revision THR for recurrent dislocation, but such liners result in restricted range of motion for the patient and have a greater prevalence of impingement of the femoral neck on the acetabular shell, leading to high stress transmission to multiple interfaces and liner damage, locking mechanism failure, dislocation, and loosening. Yet another option is the inclusion of a mobile polyethylene component between the prosthetic head and the polished inner surface of an outer metal acetabular shell, which provides a greater effective head size and improved head-to-neck ratio that can lead to improved range of motion and reduce risk of impingement and dislocation; yet, the mobile polyethylene component raises concerns regarding the potential for increased polyethylene wear or damage as well as corrosion issues at the femoral head-neck interface [9].

Thus, there remains a need in the art for a THR prosthesis that can reduce the incidence of, or prevent, hip dislocation.

## SUMMARY OF INVENTION

Aspects of the present invention relate to a hip prosthesis, methods of using the hip prosthesis, and methods of manufacturing the hip prosthesis.

The hip prosthesis comprises an acetabular component and a femoral component, in which the acetabular component comprises one or more magnets and the femoral component comprises a rotatable spherical magnet. An attractive force is generated between the one or more magnets of the acetabular component and the spherical magnet of the femoral component, which provides greater stability between the components and reduces the risk of dislocation. And because the spherical magnet is rotatable, the attractive force is maintained through the hip's range of motion. The present invention avoids the complications and limitations of conventional methods of stabilizing hip prostheses that can reduce the range of motion, increase potential for wear and damage to components, and increase taper corrosion.

Thus, in one aspect, the present invention relates to a hip prothesis comprising (a) an acetabular component comprising a shell having an approximate shape of a hollowed spherical cap that comprises a central dome, a periphery, and an intermediate wall therebetween, in which the shell comprises a concave inner surface, a convex outer surface, a thickness therebetween, and one or more magnets; and (b) a femoral component comprising (i) a stem comprising a proximal end and a distal end, (ii) a neck comprising a tapered end and a base end in which the base end of the neck is joined to the proximal end of the stem, and the neck extends at an angle from the stem, and (iii) a spherical head that is affixed to the tapered end of the neck, in which the spherical head comprises a spherical magnet and the spherical magnet is rotatable within the spherical head. The spherical head comprises a tapered volume, an outside surface, and a thickness between the tapered volume and the outside surface, in which the tapered volume is configured to receive the tapered end of the neck. The acetabular component is configured to receive all or a portion of the spherical head of the femoral component. Further, the one or more magnets of the shell of the acetabular component and the spherical magnet of the spherical head of the femoral component are oriented to generate an attractive force therebetween.

In some embodiments, the convex inner surface of the shell is configured to articulate with the outer surface of the spherical head.

In some embodiments, the acetabular component further comprises a liner that comprises a concave inner surface, a convex outer surface, and a thickness therebetween, in which the concave inner surface of the shell is configured to receive all or a portion of the convex outer surface of the liner. In certain embodiments, the convex inner surface of the liner is configured to articulate with the outer surface of the spherical head.

In some embodiments, the one or more magnets of the shell comprise a cylindrical, prism, conal, or pyramidal shape. In certain embodiments, the one or more magnets of the shell comprise a cylindrical shape. In particular embodiments, the one or more magnets of the shell comprise a cylindrical shape and have a diameter of about 2 mm to about 20 mm and a length of about 1 mm to about 15 mm; or have a diameter of about 4 mm to about 15 mm and a length of about 3 mm to about 10 mm.

In some embodiments, the one or more magnets of the shell are on the concave surface of the shell. In some embodiments, the one or more magnets of the shell are in the thickness of the shell.

In some embodiments, the one or more magnets of the shell are at or adjacent to the central dome. In certain embodiments, the one or more magnets of the shell comprise a single magnet at or adjacent to the central dome.

In some embodiments, the one or more magnets of the shell comprise a single magnet at or adjacent to the central dome, and an array of magnets surrounding the single magnet. In certain embodiments, each magnet in the array of magnets is equidistant from the single magnet, and/or each magnet in the array of magnets is equidistant from each other. The single magnet at the central dome may be oriented such that its long axis is perpendicular to the tangent line of the curvature at the central dome. In certain embodiments, the magnets in the array are oriented such that their long axes are parallel to the long axis of the single magnet. In alternative embodiments, the magnets in the array are oriented such that their long axes are angled to the long axis of the single magnet; for example, the angle between the long axis of the magnets in the array and the long axis of the single magnet may be about 10° to about 80°, or may be about 30° to about 60°.

In some embodiments, the one or more magnets of the shell comprise an array of magnets adjacent to the central dome. Each magnet in the array of magnets may be equidistant from the central dome, and/or may be equidistant from each other. In some embodiments, the magnets in the array are oriented such that their long axes are perpendicular to the tangent line of the curvature at the central dome. In alternative embodiments, the magnets in the array are oriented such that their long axes are angled to a line that is perpendicular to the tangent line of the curvature at the central dome; for instance, the angle between the long axis of the magnets in the array and the line that is perpendicular to the tangent line of the curvature at the central dome may be about 10° to about 80°, or may be about 30° to about 60°.

In some embodiments, the array of magnets comprises between 2 and 16 magnets, or between 2 and 8 magnets, or between 2 and 6 magnets, or 4 magnets.

In some embodiments, the one or more magnets of the shell comprise a magnetic material of a rare-earth magnet. In certain embodiments, the one or more magnets of the shell comprise a magnetic material of an alloy of neodymium, iron, and boron.

In some embodiments, the one or more magnets of the shell are magnetized along the long axis of the magnets.

In some embodiments, the one or more magnets of the shell comprise a casing that encloses the magnetic material.

In embodiments of the invention, the tapered volume of the spherical head extends inward from the outside surface of the spherical head. In some embodiments, the tapered volume of the spherical head is defined by an end surface that is the innermost surface of the tapered volume, and walls that extend from the outside surface to the end surface. In certain embodiments, the cross-sectional area of the tapered volume of the spherical head decreases towards the end surface from the outside surface of the spherical head. In particular embodiments, the spherical magnet of the spherical head is at or adjacent to the end surface of the tapered volume.

In some embodiments, the spherical head further comprises a casing surrounding the spherical magnet, and wherein the spherical magnet is rotatable within the casing.

In some embodiments, the spherical magnet of the spherical head comprise a diameter of about 1 mm to about 30 mm, or a diameter of about 5 mm to about 20 mm.

In some embodiments, the spherical magnet of the spherical head comprises a magnetic material of a rare-earth magnet. In certain embodiments, the spherical magnet of the spherical head comprises a magnetic material of an alloy of neodymium, iron, and boron.

In preferred embodiments, the spherical magnet of the spherical head is magnetized through its diameter.

In one aspect, the present invention relates to the hip prosthesis of the present invention for use in (i) treating a subject in need of THR; (ii) stabilizing a THR prosthesis in a subject; (iii) reducing incidence of THR dislocation in a subject, such as THR dislocation due to impingement; (iv) reducing risk of THR dislocation in a subject, such as THR dislocation due to impingement; (v) reducing risk of impingement associated with THR in a subject; (vi) reducing risk of THR subluxation in a subject; and/or (vii) reducing osteolysis associated with THR in a subject. The use may comprise implanting the hip prosthesis in the subject. In some embodiments, implantation of the hip prosthesis may comprise (a) removing all or a portion of the cartilage of the acetabulum and implanting the acetabular component; and (b) cutting the proximal femoral neck and implanting the femoral component. In certain embodiments, the hip prosthesis stabilizes the hip joint during healing after implantation.

In another aspect, the present invention relates to methods of (i) treating a subject in need of THR; (ii) stabilizing a THR prosthesis in a subject; (iii) reducing incidence of THR dislocation in a subject, such as THR dislocation due to impingement; (iv) reducing risk of THR dislocation in a subject, such as THR dislocation due to impingement; (v) reducing risk of impingement associated with THR in a subject; (vi) reducing risk of THR subluxation in a subject; and/or (vii) reducing osteolysis associated with THR in a subject. The methods may comprise implanting the hip prosthesis in the subject. In some embodiments, implantation of the hip prosthesis may comprise (a) removing all or a portion of the cartilage of the acetabulum and implanting the acetabular component; and (b) cutting the proximal femoral neck and implanting the femoral component. In certain embodiments, the hip prosthesis stabilizes the hip joint during healing after implantation.

In yet another aspect, the present invention relates to use of the hip prosthesis of the present invention for (i) treating a subject in need of THR; (ii) stabilizing a THR prosthesis in a subject; (iii) reducing incidence of THR dislocation in a subject, such as THR dislocation due to impingement; (iv) reducing risk of THR dislocation in a subject, such as THR dislocation due to impingement; (v) reducing risk of impingement associated with THR in a subject; (vi) reducing risk of THR subluxation in a subject; and/or (vii) reducing osteolysis associated with THR in a subject. The use may comprise implanting the hip prosthesis in the subject. In some embodiments, implantation of the hip prosthesis may comprise (a) removing all or a portion of the cartilage of the acetabulum and implanting the acetabular component; and (b) cutting the proximal femoral neck and implanting the femoral component. In certain embodiments, the hip prosthesis stabilizes the hip joint during healing after implantation.

A further aspect of the present invention relates to the femoral component of a hip prosthesis. The femoral component may comprise (a) a stem comprising a proximal end and a distal end; (b) a neck comprising a tapered end and a base end, in which the base end of the neck is joined to the proximal end of the stem, and the neck extends at an angle from the stem; and (c) a spherical head that is affixed to the tapered end of the neck, in which the spherical head comprises a spherical magnet, and the spherical magnet is rotatable within the spherical head. In some embodiments, the spherical head comprises a tapered volume, an outside surface, and a thickness between the tapered volume and the outside surface, in which the tapered volume is configured to receive the tapered end of the neck.

## BRIEF DESCRIPTION OF THE DRAWING FIGURES

The present disclosure will be further explained with reference to the attached drawing figures, wherein like structures are referred to by like numerals throughout the several views. The drawing figures shown are not necessarily to scale, with emphasis instead generally being placed upon illustrating the principles of the present disclosure, and some features may be exaggerated to show details of particular components. In addition, any measurements, specifications, and the like shown in the drawing figures, or described below, are intended to be illustrative, and not restrictive. Therefore, specific structural and functional details disclosed herein are not to be interpreted as limiting, but merely as a representative basis for teaching one skilled in the art to variously employ the magnetic devices and methods of their use.

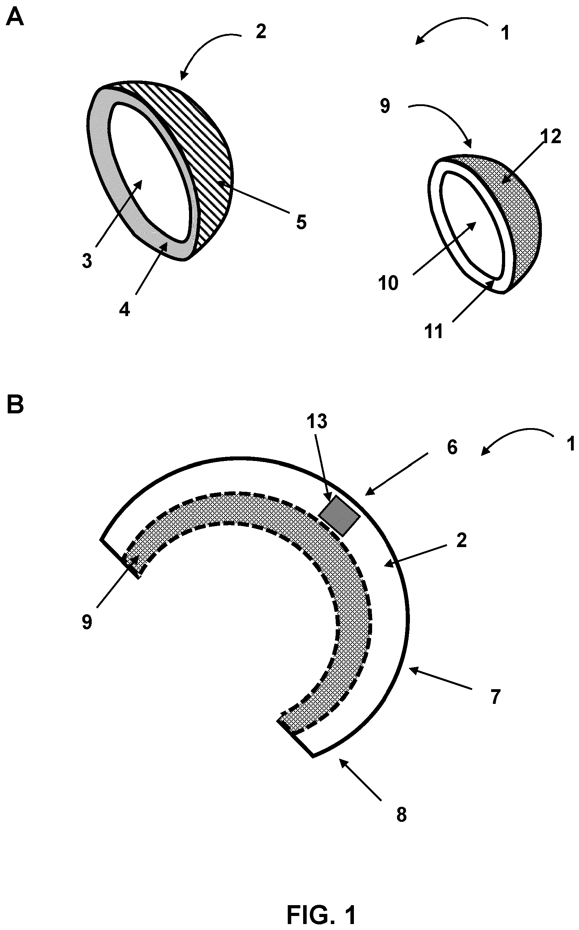

FIGS. 1A and 1B show the acetabular component of the hip prosthesis, according to embodiments of the invention. FIG. 1A shows an exploded view of the acetabular component comprising a shell and a liner, and FIG. 1B shows a cut-away view of the acetabular component.

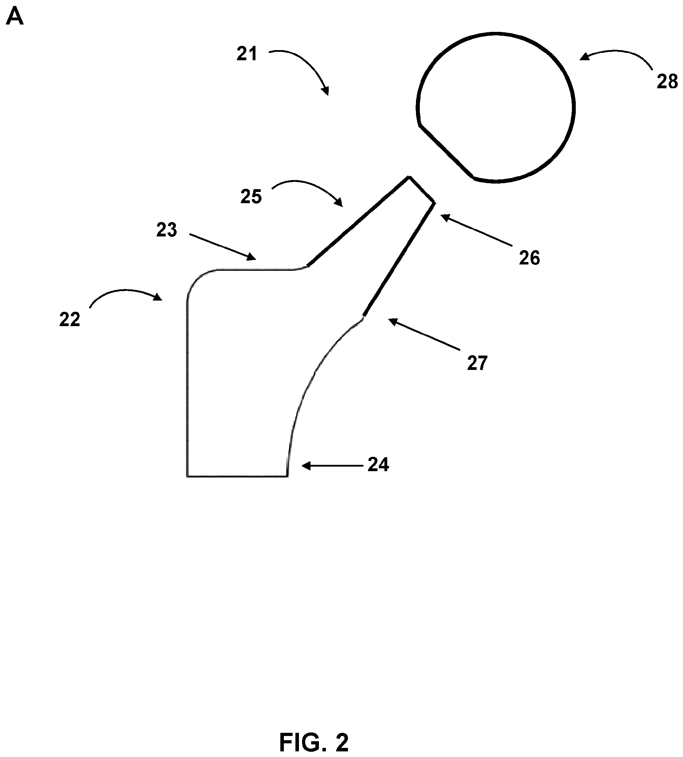

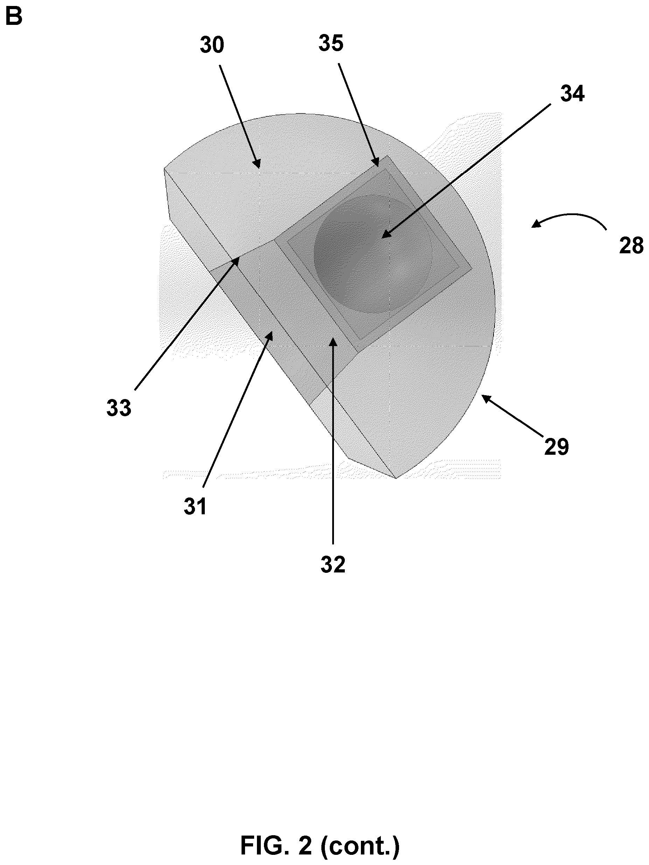

**FIGS. 2A** and **2B** show the femoral component of the hip prosthesis, according to embodiments of the invention. **FIG. 2A** shows an exploded perspective view of the femoral component, and **FIG. 2B** shows a cut-away view of the spherical head of the femoral component.

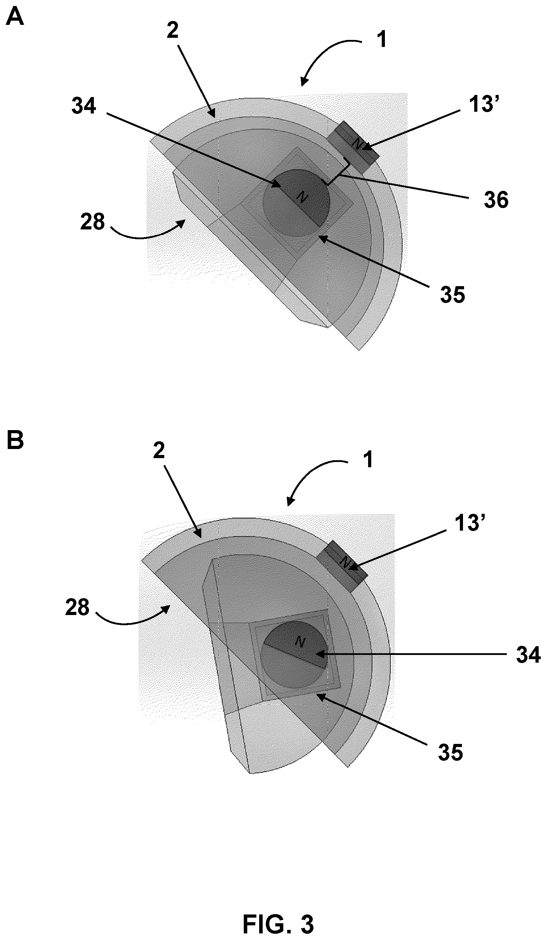

**FIGS. 3A** and **3B** show the acetabular component and femoral component according to embodiments of the invention when assembled for use, such as when implanted into a subject, in which the acetabular component comprises a single cylindrical magnet at the central dome of the shell, and the femoral component comprises a spherical magnet in the spherical head. **FIG. 3A** shows the acetabular component and the femoral component at the 0° hip position, and **FIG. 3B** shows the acetabular component and the femoral component at the 35° hip position.

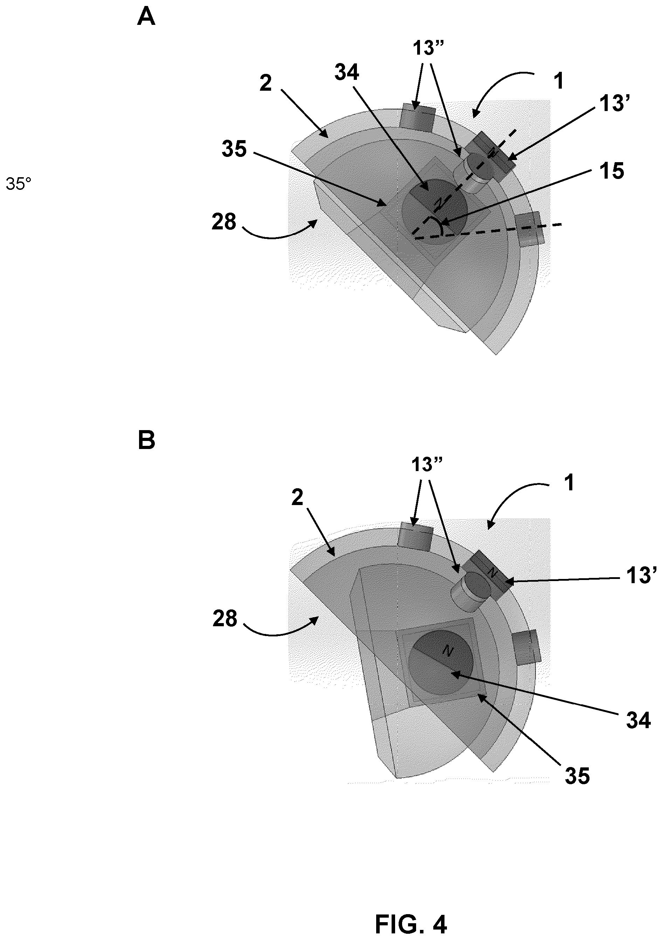

**FIGS. 4A** and **4B** show the acetabular component and femoral component according to embodiments of the invention when assembled for use, such as when implanted into a subject, in which the acetabular component comprises a single cylindrical magnet at the central dome of the shell and an array of cylindrical magnets surrounding the single magnet, and the femoral component comprises a spherical magnet in the spherical head. **FIG. 4A** shows the acetabular component and the femoral component at the 0° hip position, and **FIG. 4B** shows the acetabular component and the femoral component at the 35° hip position.

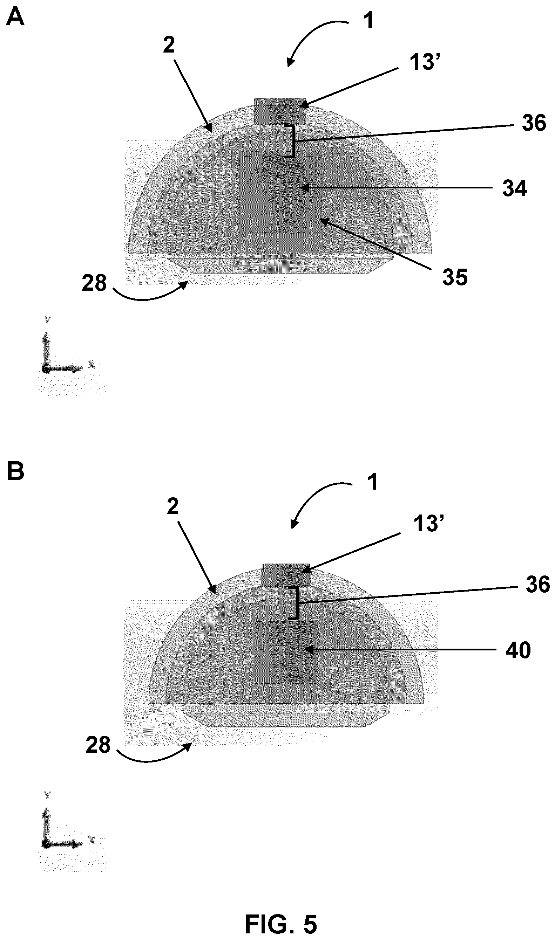

**FIGS. 5A** and **5B** show the acetabular component and femoral component according to embodiments of the invention that were modeled in Example 1, in which the acetabular component and the femoral component were at the 0° hip position, the acetabular component comprises a single cylindrical magnet at the central dome of the shell, and the femoral component comprises either a spherical magnet in the spherical head **(FIG. 5A)** or a cylindrical magnet in the spherical head **(FIG. 5B)**.

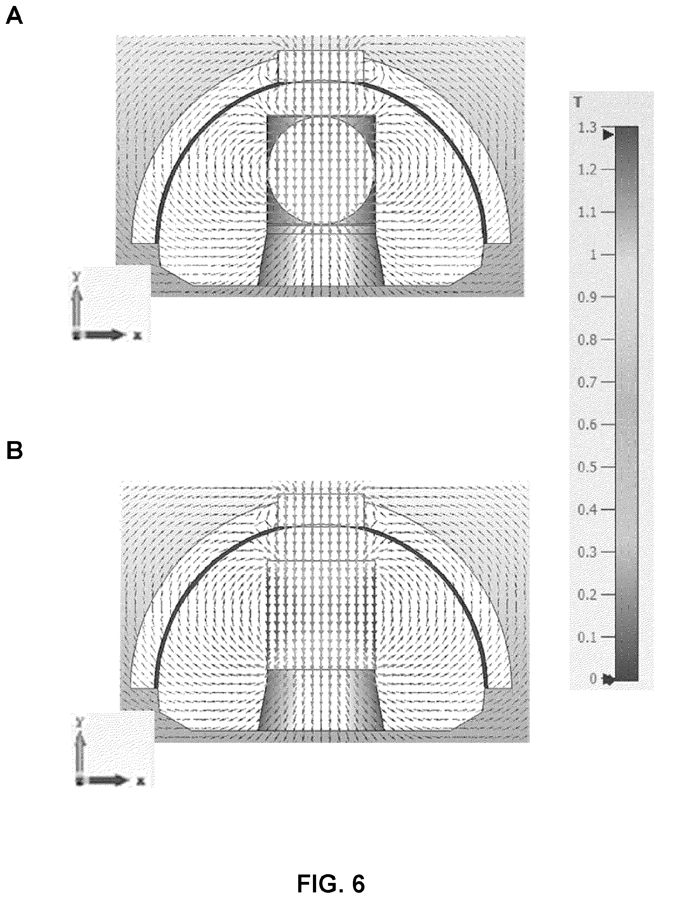

**FIGS. 6A** and **6B** shows magnetic field flux density maps generated from the modeling described in Example 1, in which the acetabular component and the femoral component were at the 0° hip position, the acetabular component comprises a single cylindrical magnet at the central dome of the shell, and the femoral component comprises either a spherical magnet in the spherical head **(FIG. 6A)** or a cylindrical magnet in the spherical head **(FIG. 6B)**. Magnetic flux density is represented by arrows that depict both the strength and direction of a magnetic field at a given point. The arrow length and width are proportional to the magnitude of the flux density in the area. Magnetic field flux lines emanate from a magnetic north pole and loop around to the south pole.

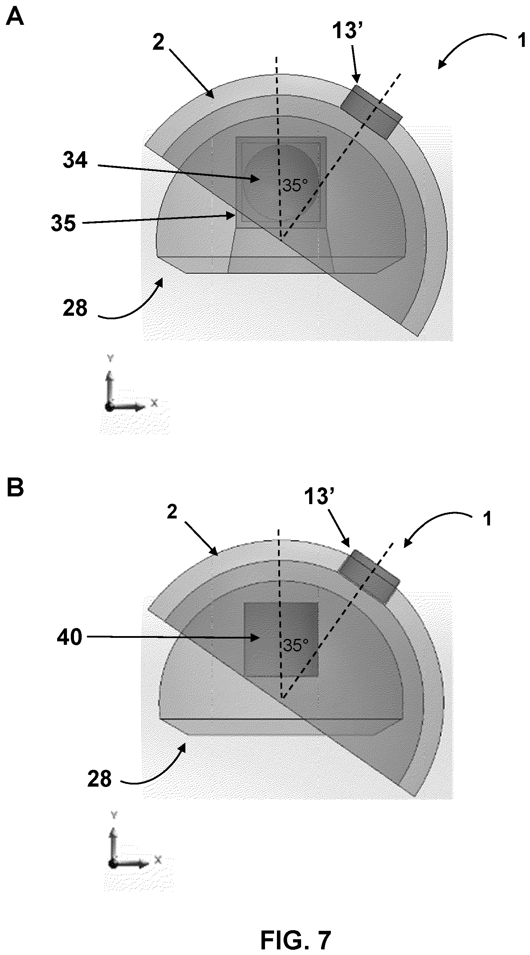

**FIGS. 7A** and **7B** show the acetabular component and femoral component according to embodiments of the invention that were modeled in Example 2, in which the acetabular component and the femoral component were at the 35° hip position, the acetabular component comprises a single cylindrical magnet at the central dome of the shell, and the femoral component comprises either a spherical magnet in the spherical head **(FIG. 7A)** or a cylindrical magnet in the spherical head **(FIG. 7B)**.

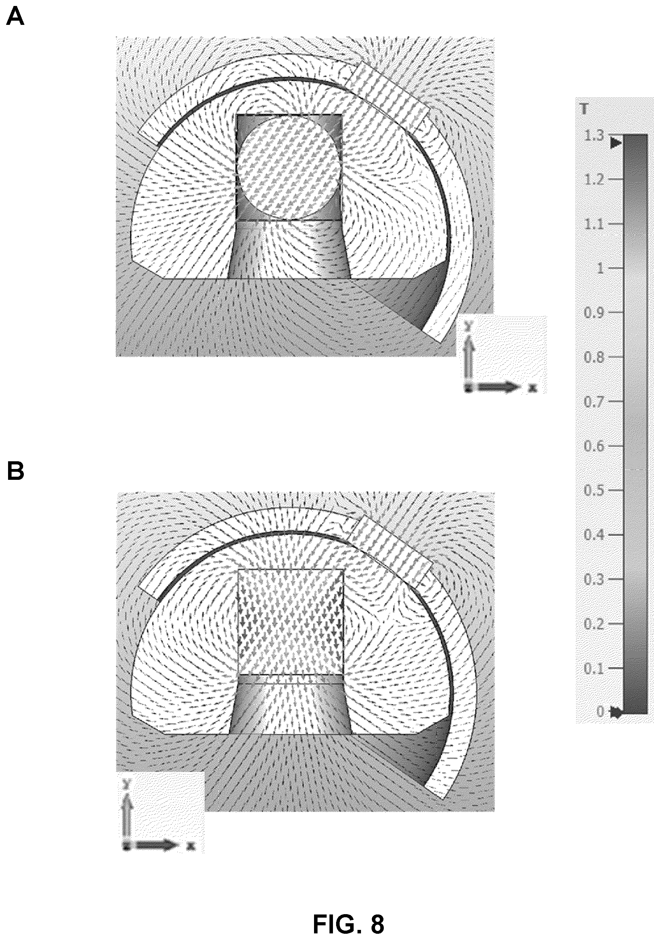

**FIGS. 8A** and **8B** shows magnetic field flux density maps generated from the modeling described in Example 2, in which the acetabular component and the femoral component were at the 35° hip position, the acetabular component comprises a single cylindrical magnet at the central dome of the shell, and the femoral component comprises either a spherical magnet in the spherical head **(FIG. 8A)** or a cylindrical magnet in the spherical head **(FIG. 8B)**. Magnetic flux density is represented by arrows that depict both the strength and direction of a magnetic field at a given point. The arrow length and width are proportional to the magnitude of the flux density in the area.

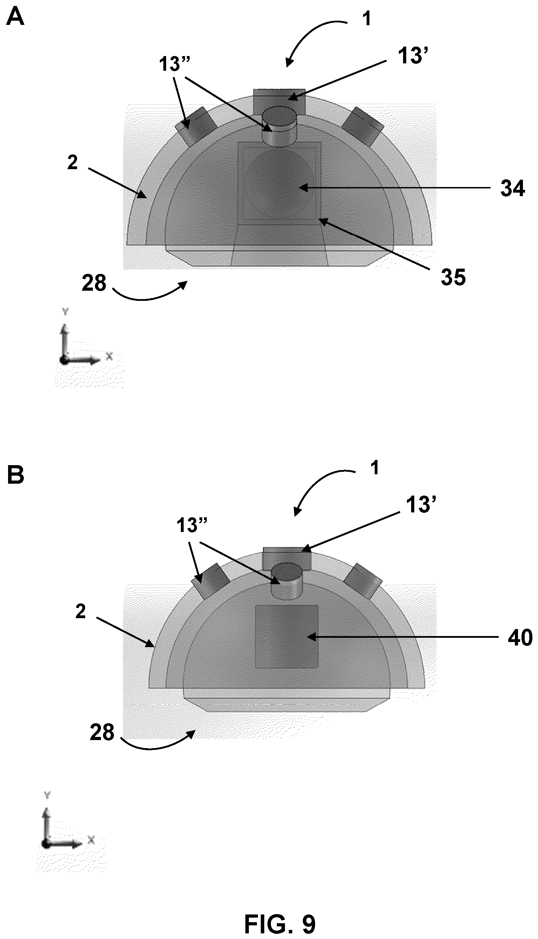

**FIGS. 9A** and **9B** show the acetabular component and femoral component according to embodiments of the invention that were modeled in Example 3, in which the acetabular component and the femoral component were at the 0° hip position, the acetabular component comprises a single cylindrical magnet at the central dome of the shell and an array of cylindrical magnets surrounding the single magnet, and the femoral component comprises either a spherical magnet in the spherical head **(FIG. 9A)** or a cylindrical magnet in the spherical head **(FIG. 9B)**.

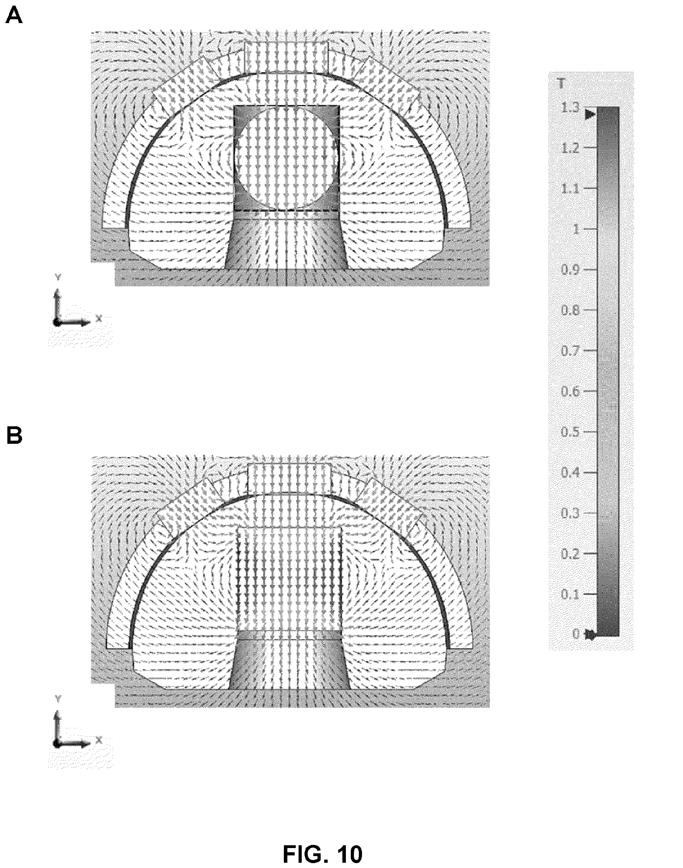

**FIGS. 10A** and **10B** shows magnetic field flux density maps generated from the modeling described in Example 3, in which the acetabular component and the femoral component were at the 0° hip position, the acetabular component comprises a single cylindrical magnet at the central dome of the shell and an array of cylindrical magnets surrounding the single magnet, and the femoral component comprises either a spherical magnet in the spherical head **(FIG. 10A)** or a cylindrical magnet in the spherical head **(FIG. 10B)**. Magnetic flux density is represented by arrows that depict both the strength and direction of a magnetic field at a given point. The arrow length and width are proportional to the magnitude of the flux density in the area.

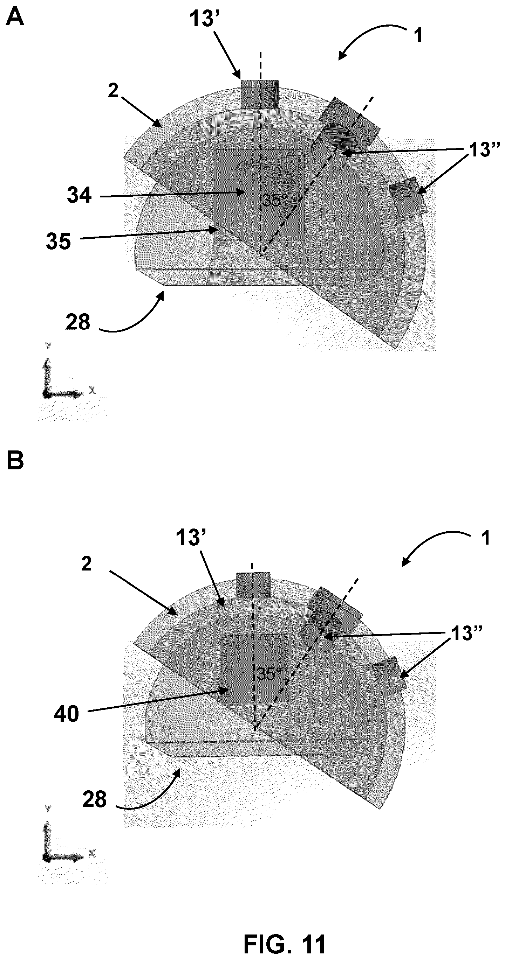

**FIGS. 11A** and **11B** show the acetabular component and femoral component according to embodiments of the invention that were modeled in Example 4, in which the acetabular component and the femoral component were at the 35° hip position, the acetabular component comprises a single cylindrical magnet at the central dome of the shell and an array of cylindrical magnets surrounding the single magnet, and the femoral component comprises either a spherical magnet in the spherical head **(FIG. 11A)** or a cylindrical magnet in the spherical head **(FIG. 11B).**

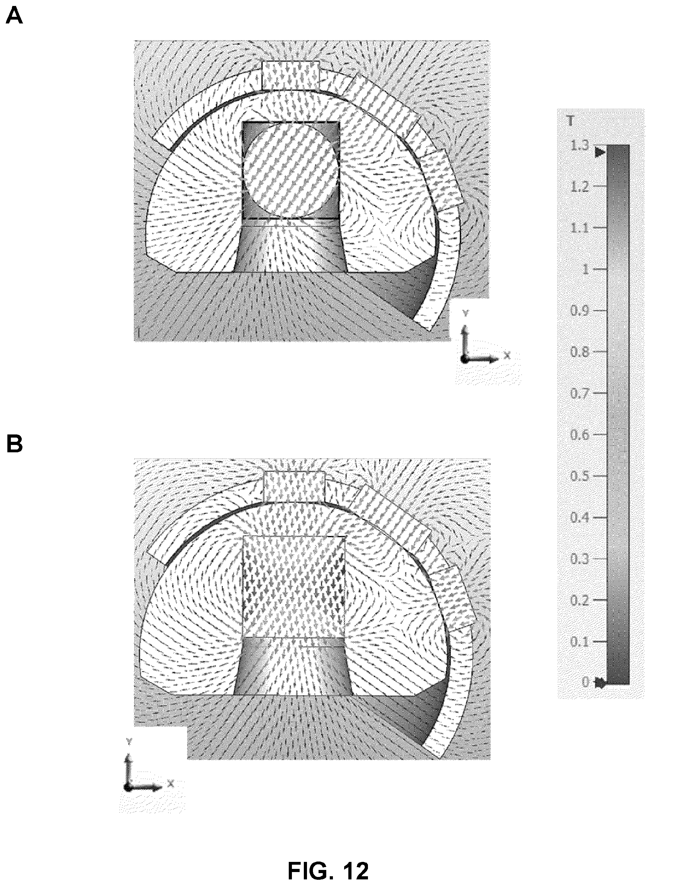

**FIGS. 12A** and **12B** shows magnetic field flux density maps generated from the modeling described in Example 4, in which the acetabular component and the femoral component were at the 35° hip position, the acetabular component comprises a single cylindrical magnet at the central dome of the shell and an array of cylindrical magnets surrounding the single magnet, and the femoral component comprises either a spherical magnet in the spherical head **(FIG. 12A)** or a cylindrical magnet in the spherical head **(FIG. 12B).** Magnetic flux density is represented by arrows that depict both the strength and direction of a magnetic field at a given point. The arrow length and width are proportional to the magnitude of the flux density in the area.

## DETAILED DESCRIPTION OF THE INVENTION

The practice of the present invention can employ, unless otherwise indicated, conventional techniques of orthopedics, biomedical engineering, tissue biology, cell biology, osteology, biomaterials, and clinical practice, which are within the skill of the art.

In order that the present invention can be more readily understood, certain terms are first defined. Additional definitions are set forth throughout the disclosure. Unless defined otherwise, all technical and scientific terms used herein have the same meaning as commonly understood by one of ordinary skill in the art to which this invention is related.

Any headings provided herein are not limitations of the various aspects or embodiments of the invention, which can be had by reference to the specification as a whole. Accordingly, the terms defined immediately below are more fully defined by reference to the specification in its entirety.

All references cited in this disclosure are hereby incorporated by reference in their entireties. In addition, any manufacturers' instructions or catalogues for any products cited or mentioned herein are incorporated by reference. Documents incorporated by reference into this text, or any teachings therein, can be used in the practice of the present invention. Documents incorporated by reference into this text are not admitted to be prior art.

### Definitions

The phraseology or terminology in this disclosure is for the purpose of description and not of limitation, such that the terminology or phraseology of the present specification is to be interpreted by the skilled artisan in light of the teachings and guidance.

As used in this specification and the appended claims, the singular forms "a," "an," and "the" include plural referents, unless the context clearly dictates otherwise. The terms "a" (or "an") as well as the terms "one or more" and "at least one" can be used interchangeably.

Furthermore, "and/or" is to be taken as specific disclosure of each of the two specified features or components with or without the other. Thus, the term "and/or" as used in a phrase such as "A and/or B" is intended to include A and B, A or B, A (alone), and B (alone). Likewise, the term "and/or" as used in a phrase such as "A, B, and/or C" is intended to include A, B, and C; A, B, or C; A or B; A or C; B or C; A and B; A and C; B and C; A (alone); B (alone); and C (alone).

Wherever embodiments are described with the language "comprising," otherwise analogous embodiments described in terms of "consisting of" and/or "consisting essentially of" are included.

Units, prefixes, and symbols are denoted in their Système International de Unites (SI) accepted form. Numeric ranges are inclusive of the numbers defining the range, and any individual value provided herein can serve as an endpoint for a range that includes other individual values provided herein. For example, a set of values such as 1, 2, 3, 8, 9, and 10 is also a disclosure of a range of numbers from 1-10, from 1-8, from 3-9, and so forth. Likewise, a disclosed range is a disclosure of each individual value (i.e., intermediate) encompassed by the range, including integers and fractions. For example, a stated range of 5-10 is also a disclosure of 5, 6, 7, 8, 9, and 10 individually, and of 5.2, 7.5, 8.7, and so forth.

Unless otherwise indicated, the terms "at least" or "about" preceding a series of elements is to be understood to refer to every element in the series. The term "about" preceding a numerical value includes ± 10% of the recited value. For example, a concentration of about 1 mg/mL includes 0.9 mg/mL to 1.1 mg/mL. Likewise, a concentration range of about 1% to 10% (w/v) includes 0.9% (w/v) to 11% (w/v).

A "subject" or "individual" or "animal" or "patient" or "mammal," is any subject, particularly a mammalian subject, for whom diagnosis, prognosis, or therapy is desired. Mammalian subjects include humans, domestic animals, farm animals, sports animals, and laboratory animals including, e.g., humans, non-human primates, canines, felines, porcines, bovines, equines, rodents, including rats and mice, rabbits, etc. In some embodiments the subjects are human.

Terms such as "treating" or "treatment" or "to treat" or "alleviating" or "to alleviate" refer to therapeutic measures that cure, slow down, lessen symptoms of, and/or halt progression of a diagnosed pathologic condition or disorder. In certain embodiments, a subject is successfully "treated" for a disease or disorder if the patient shows total, partial, or transient alleviation or elimination of at least one symptom or measurable physical parameter associated with the disease or disorder.

The terms "inhibit," "block," "reduce," and "suppress" are used interchangeably and refer to any statistically significant decrease in occurrence or activity, including full blocking of the occurrence or activity. For example, "inhibition" can refer to a decrease of about 10%, 20%, 30%, 40%, 50%, 60%, 70%, 80%, 90% or 100% in activity or occurrence.

**Hip Prosthesis**

The THR prosthesis of the present invention comprises an acetabular component and a femoral component.

*Acetabular Component*

As shown in **FIGS. 1A** and **1B,** the acetabular component **1** of the hip prosthesis comprises a shell **2** and optionally a liner **9.** The shell **2** comprises a concave inner surface **3,** a convex outer surface **5,** and a thickness **4** between the inner surface **3** and the outer surface **5.** The shell **2** has an approximate shape of a hollowed spherical cap comprising a central dome **6,** a periphery **8,** and an intermediate wall **7** therebetween.

As used herein, a "spherical cap" refers to a portion of a sphere cut off by a plane. In some embodiments, the spherical cap may be full hemispherical. In other embodiments, the spherical cap may be partial-hemispherical.

As used herein, an "approximate shape" means that the shell **2** may have a shape that is not a perfect spherical cap, *i.e.,* it may not be based on a perfect sphere. For example, the spherical cap may not be based on a sphere in which each radius is equal in length; rather there may be some variation in the lengths of each radius, such as up to 10% variation, or up to 5% variation.

In some embodiments, the shell **2** has a shape of a hollowed spherical cap comprising a central dome **6,** a periphery **8,** and an intermediate wall **7** therebetween.

As used herein, a "central dome" of a shape or approximate shape of a spherical cap refers to a portion of the curved surface of the spherical cap that is equidistance to the edge of the base.

The liner **9** comprises a concave inner surface **10,** a convex outer surface **12,** and a thickness **11** between the inner surface **10** and the outer surface **12.** The liner **9** is configured to fit within the shell **2,** such that the outer surface **12** of the liner **9** is adjacent to the inner surface **3** of the shell **2.** In some embodiments, the liner **9** may be a constrained liner.

The acetabular component **1** may comprise materials known in the art for acetabular components of THR prostheses. For instance, the acetabular component **1** may comprise a metal such as stainless steel, titanium, chromium, cobalt, or a combination thereof; a plastic such as polyethylene or cross-linked polyethylene; or a ceramic. Notably, the shell **2** and liner **9** may comprise different materials. For example, the shell **2** may comprise a metal while the liner **9** may comprise a plastic or ceramic.

The shell **2** comprises one or more magnets **13.** The one of more magnets may be within the thickness **4** of the shell **2,** on the inner surface **3** of the shell **2,** on the outer surface **5** of the shell **2,** embedded in the inner surface **3** of the shell **2,** embedded in the outer surface **5** of the shell **2,** or a combination thereof (*e.g.*, if the shell **2** comprises more than one magnet **13,** one magnet **13** may be within the thickness **4** of the shell **2,** another magnet **13** may be embedded on the inner surface **3** of the shell **2,** *etc.*). In embodiments in which the acetabular component **1** comprises the liner **9,** one or more magnets **13** may be on, or embedded in, the inner surface **3** of the shell 2, such that the one or more magnets **13** are between the inner surface **3** of the shell **2** and the outer surface **12** of the liner **9.** In preferred embodiments, the one or more magnets **13** are within the thickness of the shell **2.**

The one or more magnets **13** may be positioned in the shell **2** at or adjacent to the central dome **6,** at the periphery **8,** in the intermediate wall **7,** or a combination thereof. In some embodiments, the one of more magnets **13** are positioned at or adjacent to the central dome **6** of the shell **2.**

In certain embodiments, a single magnet **13'** is positioned at the central dome **6** of the shell **2.** An example of such positioning of the single magnet **13'** is shown in **FIGS. 3A** and **3B.** In certain embodiments, an array of magnets **13"** is positioned at or adjacent to the central dome **6** of the shell **2.** As used herein, an "array of magnets" refers to a group of two or more magnets. Each magnet in the array of magnets **13"** may be equidistant from each other. In embodiments in which the array of magnets **13"** is positioned adjacent to the central dome **6** of the shell **2,** each magnet in the array of magnets **13"** may be equidistant from the central dome **6.**

In some embodiments, a single magnet **13'** is positioned at the central dome **6** of the shell **2,** and an array of magnets **13"** is positioned to surround the single magnet **13'.** In certain embodiments, each magnet in the array of magnets **13"** may be equidistant from each other. In certain embodiments, each magnet in the array of magnets **13"** may be equidistant to the single magnet **13'**. In such embodiments, the magnets in the array of magnets **13"** may also be distributed evenly around the circumference of the shell **2.** An example of such positioning of the single magnet **13'** and the array of magnets **13"** is shown in **FIGS. 4A** and **4B.**

In some embodiments, the array of magnets **13"** may comprise 2 to 16 magnets, or 2 to 12 magnets, or 4 to 12 magnets, or 2 to 10 magnets, or 4 to 10 magnets, or 2 to 8 magnets, or 4 to 8 magnets, or 2 to 6 magnets, such as 2 magnets, or 3 magnets, or 4 magnets, or 5 magnets, or 6 magnets, or 7 magnets, or 8 magnets, or 9 magnets, or 10 magnets, or 11 magnets, or 12 magnets, or 13 magnets, or 14 magnets, or 15 magnets, or 16 magnets.

In embodiments in which the shell **2** comprises a single magnet **13'** at the central dome, the single magnet **13'** may be oriented such that the long axis of the magnet is perpendicular to the tangent line of the curvature at the central dome **6.** Such an orientation is shown in **FIGS. 3A** and **3B.** In some embodiments, the single magnet **13'** may be oriented such that the long axis of the magnet is perpendicular to the tangent line of the curvature of the inner surface **3** of the shell **2** at the central dome **6.** In certain embodiments in which the acetabular component **1** comprises a liner **6,** the single magnet **13'** may be oriented such that the long axis of the magnet is perpendicular to the tangent line of the curvature of the inner surface 10 of the liner **9** at the central dome **6.**

In embodiments in which an array of magnets **13"** surround the single magnet **13',** the magnets in the array of magnets **13"** may be oriented such that their long axes are parallel to the long axis of the single magnet **13'.** Alternatively, the magnets in the array of magnets **13"** may be oriented such that their long axes are angled to the long axis of the single magnet **13',** as demonstrated in **FIG. 4A** and **4B.** This angle **15** may be about 10° to about 80°, or about 20° to about 70°, or about 30° to about 60°, or about 30° to about 50°, or about 30° to about 40°, or any angle therebetween, such as about 35°, or about 40°, or about 45°, or about 50°.

In embodiments in which an array of magnets **13"** is positioned at or adjacent to the central dome **6** of the shell **2,** the magnets in the array of magnets **13"** may be oriented such that their long axes are perpendicular to the tangent line of the curvature at the central dome **6.** Alternatively, the magnets in the array of magnets **13"** may be oriented such that their long axes are angled to a line that is perpendicular to the tangent line of the curvature at the central dome **6.** This angle may be about 10° to about 80°, or about 20° to about 70°, or about 30° to about 60°, or any angle therebetween, such as about 35°, or about or about 40°, or about or about 45°, or about 50°.

The one or more magnets **13** may be within the thickness **4** of the shell **2,** on the inner surface **3** of the shell **2,** on the outer surface **5** of the shell **2,** embedded in the inner surface **3** of the shell **2,** or embedded in the outer surface **5** of the shell **2.**

The magnet(s) may be generally any geometric shape, such as a cylinder, prism (including rectangular prism, hexagonal prism, triangular prism, cube, *etc.),* cone, spherical, and pyramid. In certain embodiments, the one or more magnets are cylindrical. If more than one magnet is present, each magnet may comprise the same or a different geometric shape. In preferred embodiments, each magnet comprises a cylindrical shape.

In embodiments in which the magnet is cylindrical, the "long axis" refers to the line that is formed by the centers of the circular bases of the cylinder. In embodiments in which the magnet is prism-shaped, the "long axis" refers to the line formed by the centers of bases in the direction of the longest dimension of the magnet. In embodiments in which the magnet is conal or pyramidal, the "long axis" refers to the line between the apex and the center of the base and perpendicular to the base.

In some embodiments, a surface of the magnet may comprise a geometric contour, such as a curvature, which is similar to the curvature of the shell and/or the curvature of the liner.

The magnet(s) may comprise magnetic materials known in the art. For example, the magnetic materials may be iron-based, nickel-based, cobalt-based, or an alloy of rare-earth metals. In some embodiments, the magnetic material may be a rare-earth magnet, which generally has strong attraction and repulsion forces, and has high retentive capacity and resistance to demagnification. In certain embodiments, the rare-earth magnet is an alloy of neodymium, iron, and boron ("NdFeB"). NdFeB magnets may provide strong permanent magnetism, high retentive capacity, and resistance to demagnetization. In preferred embodiments, the magnetic material is a N52 NdFeB rare earth magnet.

In embodiments of the invention, the one or more magnets of the acetabular component are magnetized along the long axis of the magnet(s). For example, in embodiments in which the magnet(s) are generally cylindrical, the magnet(s) are magnetized along the axis that passes through the center of each circular end of the magnet. Alternatively, the one or more magnets of the acetabular component are magnetized in a radial orientation. In certain embodiments, magnet(s) at the central dome are magnetized axially along the length of the long axis of the magnet(s), and magnet(s) in an array surrounding the magnet(s) at central dome are magnetized axially along the length of the long axis of the magnet(s) or are magnetized in a radial orientation. In embodiments in which the magnet(s) are spherical, they magnet(s) may be magnetized through their diameter.

In some embodiments, each magnet may be enclosed by a casing that prevents the magnet from exposure to the environment. The magnet may be hermetically sealed within the casing. In some embodiments, the casing may comprise two or more components (e.g., an upper component and a lower component), in which the two or more components may be attached together (*e.g.*, by laser-welding) in order to create a hermetically-sealed environment for the magnetic material.

The casing may be fabricated with a metal alloy known in the art for orthopedic applications, for example, titanium, cobalt chromium, or stainless steel. In certain embodiments, the casing or plate may comprise a polymer, such as polyetheretherketone (PEEK) or polyurethane, or a combination thereof. In alternative embodiments, the casing or plate may comprise composites of polymers and fibers, such as carbon fiber-reinforced PEEK.

The shape of the casing may be primarily determined by the shape of the magnet within the casing. In some embodiments, the casing may comprise the same general shape as the magnet. For example, if the magnet is generally cylindrical, the casing may also be generally cylindrical. In some embodiments, the casing may be in the form of a screw, which may be compatible for use with the magnet that is generally cylindrical, prism-shaped, conal, or pyramidal.

The casing may comprise an exterior surface that faces the outer environment, and an interior surface that faces the magnet. The exterior surface may be smooth or may comprise surface modifications that stabilize and/or prevent movement, such as rotation, of the magnet positioned in the acetabular component. In some embodiments, the surface modifications may adhere the casing to the shell **2** or may generate friction between the casing and the shell **2.** The surface modifications may comprise a roughened surface or a pattern of protrusions that are raised from the surface. The surface modifications may also comprise screw thread(s) or a grooved design, such as in embodiments in which the casing is in the form of a screw, or any other acceptable surgical configuration.

The one or more magnets **13,** or casing(s) containing the one or more magnets **13,** may be fixed in placed in the shell **2** by means known in the art, including, but not limited to, screw designs, taper locks, and press fit.

The magnet may comprise a size appropriate for use in the acetabular component of a hip prosthesis and for generating the desired magnetic force to prevent dislocation between the acetabular component and the femoral component. For example, in embodiments in which the magnets are generally cylindrical, the magnets may have a diameter of about 2 mm to about 20 mm, or about 3 mm to about 18 mm, or about 4 mm to about 15 mm, or any diameter therebetween; and a length of about 1 mm to about 15 mm, or about 3 mm to about 10 mm, or any length therebetween. In some embodiments, the size of the cylindrical magnet may depend on whether the magnet is positioned at the dome of the acetabular component or at the intermediate walls of the acetabular component. For example, a single cylindrical magnet **13'** positioned at the central dome **6** of the shell **2** may have a diameter of about 4 mm to about 18 mm, or about 6 mm to about 14 mm, or any diameter therebetween; and cylindrical magnets positioned adjacent to the central dome **6** or that surround a single magnet **13'** at the central dome **6** may have a diameter of about 3 mm to about 15 mm, or about 5 mm to about 10 mm, or any diameter therebetween.

In embodiments in which the shell **2** comprises a single magnet **13'** that is a larger magnet, *i.e.,* about 15 mm to about 20 mm in diameter and/or about 10 mm to about 15 mm in length, the curvature of the outer surface 5 may be flattened at the central dome **6** to accommodate the larger central magnet **13'.** The flattened curvature may be accompanied by a reduced shell thickness **4** and/or, in embodiments in which the acetabular component **1** also comprises a liner **9,** a reduced liner thickness **11.**

*Femoral Component*

In embodiments of the invention, and as demonstrated in **FIGS. 2A** and **2B,** the femoral component **21** of the hip prosthesis comprises a stem portion **22** that comprises a proximal end **23** and a distal end **24;** a neck portion **25** that comprises a tapered end **26** and a base end **27;** and a spherical head **28.** The neck portion **25** extends at an angle from the stem portion **22,** in which the neck portion **25** and the stem portion **22** are joined at the base end **27** of the neck portion **25** and the proximal end **23** of the stem portion **22.** The spherical head **28** is affixed to the tapered end **26** of the neck portion **25,** and comprises a rotatable spherical magnet **34.**

The cross-sectional area of the neck portion **25** may decrease towards its tapered end **26,** such that cross-sectional area at the base end **27** is greater than the cross-sectional area at the tapered end **26.**

As shown in **FIG. 2B,** the spherical head **28** may comprise a tapered volume **31,** an outside surface **29,** and a thickness **30** between the outside surface **29** and the tapered volume **31.** The tapered volume **31** extends inward from the outside surface **29** of the spherical head **28** and is configured to receive all or part of the neck portion **25.** The tapered volume **31** is defined by an end surface **32** that is the innermost edge of the tapered volume **31,** and walls **33** that extend from the outside surface **29** to the end surface **32.** The cross-sectional area of the tapered volume **31** may decrease towards the end surface **32,** such that the cross-sectional area of the tapered volume **31** near the outside surface **29** is greater than the cross-sectional area of the tapered volume **31** at the end surface **32.**

The spherical head may not comprise the shape of a perfect sphere. For example, the spherical head may comprise a flattened side. In some embodiments, the tapered volume **31** may extend inward from the outside surface **29** at a flattened side.

The femoral component **21** may comprise materials known in the art for femoral components **21** of THR prostheses. For instance, the femoral component **21** may comprise a metal such as stainless steel, titanium, chromium, cobalt, or a combination thereof; a plastic such as polyethylene or cross-linked polyethylene; or a ceramic. Notably, the stem portion **22,** the neck portion **25,** and the spherical head **28** of the femoral component **21** may comprise different materials. For example, the stem portion **22** and the neck portion **25** may comprise a metal while the spherical head **28** may comprise a ceramic.

The spherical magnet **34** is rotatable, *i.e.,* is free to rotate within the spherical head **34.** The spherical magnet is not affixed within the spherical head such that its freedom to rotate is inhibited.

The spherical magnet **34** is magnetized through its diameter and generates an attractive force with the one or more magnet **13** of the acetabular component **1.** As illustrated in **FIGS. 3** and **4,** upon implantation of the hip prosthesis, the spherical magnet **34** will rotate and maintain an attractive force with the one or more magnet **13** of the acetabular component **1** through the range of motion, which is demonstrated in the Examples below. This occurs despite the location of the one or more magnets **13** in the acetabular component **1,** for example, when the shell **2** of the acetabular component **1** comprises a single magnet **13'** at the central dome **6** (*see* **FIGS. 3A** and **3B),** or when the shell **2** of the acetabular component **1** comprises a single magnet **13'** at the central dome **6** surrounded by an array of magnets **13"** (*see* **FIGS. 4A** and **4B).**

The spherical magnet **34** may comprise magnetic materials known in the art. For example, the magnetic materials may be iron-based, nickel-based, cobalt-based, or an alloy of rare-earth metals. In some embodiments, the magnetic material may be a rare-earth magnet, which generally has strong attraction and repulsion forces, and has high retentive capacity and resistance to demagnification. In certain embodiments, the rare-earth magnet is an alloy of NdFeB.

In preferred embodiments, the spherical magnet **34** is enclosed by a casing **35** that prevents the magnet from exposure to the environment. The spherical magnet **34** may be hermetically sealed within the casing **35.** In some embodiments, the casing **35** may comprise two or more components (*e.g.*, an upper component and a lower component), in which the two or more components may be attached together (*e.g.*, by laser-welding) in order to create a hermetically-sealed environment for the magnetic material. The spherical magnet is not affixed within the casing such that its freedom to rotate is inhibited.

The casing may be fabricated with a metal alloy known in the art for orthopedic applications, for example, titanium, cobalt chromium, or stainless steel. In certain embodiments, the casing or plate may comprise a polymer, such as PEEK or polyurethane, or a combination thereof. In alternative embodiments, the casing or plate may comprise composites of polymers and fibers, such as carbon fiber-reinforced PEEK.

In some embodiments, the casing may comprise the same general shape as the spherical head **34.** Preferably, the casing may be cylindrical, prism-shaped, conal, or pyramidal.

The casing **35** may comprise an exterior surface that faces the outer environment, and an interior surface that faces the magnet. The exterior surface may be smooth or may comprise surface modifications that stabilize and/or prevent movement, such as rotation, of the casing positioned in the spherical head **28.** In some embodiments, the surface modifications may adhere the casing **35** to the spherical head **28** or may generate friction between the casing **35** and the spherical head **28.** The surface modifications may comprise a roughened surface or a pattern of protrusions that are raised from the surface. The surface modifications may also comprise screw thread(s) or a grooved design, such as in embodiments in which the casing is in the form of a screw, or any other acceptable surgical configuration.

The spherical magnet **34** may comprise a size appropriate for use in the spherical head of a femoral component of a hip prosthesis and for generating the desired magnetic force to prevent dislocation between the acetabular component and the femoral component. For example, spherical head **34** may have a diameter of about 1 mm to about 30 mm, or about 3 mm to about 25 mm, or about 5 mm to about 20 mm, or about 10 mm to about 20 mm, or any diameter therebetween, such as 1 mm, 2 mm, 3 mm, 4 mm, 5 mm, 6 mm, 7 mm, 8 mm, 9 mm, 10 mm, 11 mm, 12 mm, 13 mm, 14 mm, 15 mm, 16 mm, 17 mm, 18 mm, 19 mm, 20 mm, 21 mm, 22 mm, 23 mm, 24 mm, 25 mm, 26 mm, 27 mm, 28 mm, 29 mm, or 30 mm.

The spherical magnet **34** may be positioned in the spherical head at the end surface **32** of the tapered volume **31,** or adjacent to the end volume **32.** In some embodiments, the spherical magnet 34 may be positioned such that, when the acetabular component and femoral component are implanted, the separation distance **36** between the spherical magnet **34** in the spherical head **28** of the femoral component **21** and a magnet **13** at the central dome **6** of the shell **2** of the acetabular component **1** when the hip position is at 0° is about 2 mm to 15 mm, or about 2 mm to 10 mm, or about 3 mm to 10 mm, or about 3 mm to 8 mm, or about 5 mm to 7 mm. As used herein, a 0° hip position is the "rest position" or "resting position," in which the leg is not abducted or adducted, not flexed or hyperextended, and not rotated relative to the hip. Such a position is consistent to when an individual is in a typical standing or laying position with the feet.

The spherical magnet **34,** or casing containing the spherical magnet **34,** may be positioned in the spherical head **28** through means known in the art. Such affixing means include, but are not limited to, press fit, screw designs (*e.g.*, a screw with the enclosed magnet is screwed into the spherical head **28),** and taper lock.

**Use of the Hip Prosthesis**

Aspects of the present invention are directed to (i) a method of treating a subject in need of THR; (ii) a method of stabilizing a THR prosthesis in a subject; (iii) a method of reducing incidence of THR dislocation in a subject, such as THR dislocation due to impingement; (iv) a method of reducing risk of THR dislocation in a subject, such as THR dislocation due to impingement; (v) a method of reducing risk of impingement associated with THR in a subject; (vi) a method of reducing risk of THR subluxation in a subject; and (vii) a method of reducing osteolysis associated with THR in a subject. Aspects of the present invention are also directed to the use of the hip prosthesis of the present invention to (i) treat a subject in need of THR; (ii) stabilize a hip prosthesis in a subject undergoing THR; (iii) reduce incidence of THR dislocation in a subject, such as THR dislocation due to impingement; (iv) reduce risk of THR dislocation in a subject, such as THR dislocation due to impingement; (v) reduce risk of impingement associated with THR in a subject, (vi) reduce risk of THR subluxation in a subject; and (vii) reduce osteolysis associated with THR in a subject. Further, aspects of the invention are directed to a hip prosthesis of the present invention for use in (i) treating a subject in need of THR; (ii) stabilizing a THR prosthesis in a subject; (iii) reducing incidence of THR dislocation in a subject, such as THR dislocation due to impingement; (iv) reducing risk of THR dislocation in a subject, such as THR dislocation due to impingement; (v) reducing risk of impingement associated with THR in a subject; (vi) reducing risk of THR subluxation in a subject; and (vii) reducing osteolysis associated with THR in a subject.

These methods or uses of the present invention may comprise implanting the hip prosthesis in the subject. Implantation of the hip prosthesis may comprise (a) removing all or a portion of the cartilage of the acetabulum and implanting the acetabular component; and (b) cutting the proximal femoral neck and implanting the femoral component. In some embodiments, implantation of the femoral component may comprise affixing all or part of the stem portion of the femoral component into the marrow cavity of the femur by, for example, press fit or using bone cement. The spherical head of the femoral component is fit into the inner surface of the shell or, if a liner is present, the inner surface of the liner.

In some embodiments, the hip prosthesis stabilizes the hip joint during healing after implantation. In certain embodiments, the healing is at the site of incision, in the surrounding tissues, or a combination thereof.

**Methods of Manufacturing the HIP Prosthesis**

An aspect of the present invention relates to methods of manufacturing the hip prosthesis of the present invention. In some embodiments, the method comprises preparing one or more bores in the shell of the acetabular component and a bore in the spherical head of the femoral component of a hip prosthesis, and inserting a magnet into each of the bores of the shell and a spherical magnet into the bore of the spherical head.

In some embodiments, the magnets inserted into the bores are enclosed in a casing in accordance with the hip prosthesis of the present invention.

In some embodiments, the acetabular component and the femoral component used in the method of manufacturing is initially an acetabular component and a femoral component as known in the art.

The one or more bores may be prepared by methods known in the art, such as using a drill or other known devices. Bores may be in a shape that accommodates the shape of the magnets that will be inserted therein; for example, a bore may be cylindrical to accommodate a cylindrical magnet.

The placement of the bores may be based on the position and orientation that is intended for the magnets that will subsequently be inserted into the bores in order to form the hip prosthesis of the present invention. For example, in embodiments for manufacturing a hip prosthesis in which the shell of the acetabular component comprises a single magnet at the central dome, a bore may be prepared at the location of the central dome and in a direction perpendicular to the tangent of the curvature at the central dome, *e.g.,* a direction in which the long axis of the bore is perpendicular to the tangent of the curvature at the central dome. In embodiments for manufacturing a hip prosthesis in which the shell additionally comprises an array of magnets surrounding the single magnet at the central dome, bores for each of the magnets in the array may be prepared surrounding the bore prepared for the single magnet. These bores may be prepared through the outer surface of the shell, through the inner surface of the shell, through the outer surface of the liner, or a combination thereof. In certain embodiments, bores may be prepared in both the inner surface of the shell and the outer surface of the liner, in which the magnets after insertion will extend between the shell and the liner; such bores may be cylindrical or, alternatively, may be in the shape of a groove that accommodates a portion of a magnet.

In some embodiments, a bore may be prepared in the femoral component at the location of the end surface of the spherical head in a direction perpendicular to the end surface, *e.g.,* a direction in which the long axis of the bore is perpendicular to the end surface. The bore may be prepared through the tapered volume, such as through the end surface, through the walls, or a combination thereof. Alternatively, or in addition, the bore may be prepared through the outside surface of the spherical head.

In embodiments in which the magnet is cylindrical, the "long axis" of the bore refers to the line that is formed by the centers of the circular bases of the cylinder. In embodiments in which the bore is another shape such as a prism-shaped, the "long axis" refers to the line formed by the centers of bases in the direction of the longest dimension of the bore. In embodiments in which the bore is conal or pyramidal, the "long axis" refers to the line between the apex and the center of the base and perpendicular to the base.

In an aspect of the invention, the method of manufacturing the hip prosthesis of the present invention does not include preparing the bores; rather the method comprises inserting magnets into bores within the shell of the acetabular component and the spherical head of the femoral component of the prosthesis, in which the bores are positioned and oriented as described herein for the magnets in the hip prosthesis that comprises magnets according to embodiments of the present invention.

With this in mind, an aspect of the invention relates to a hip prosthesis that can accommodate magnets. The hip prosthesis may comprise an acetabular component comprising a shell with one or more bores, and a femoral component comprising a spherical head with one. These bores may be positioned and oriented as described herein for the magnets in the hip prosthesis that comprises magnets according to embodiments of the present invention (*e.g.*, bores positioned at the central dome of the shell of the acetabular component, surrounding the central dome of the shell of the acetabular component, at end the surface in the spherical head of the femoral component, *etc.*).

The magnets may be inserted into the bores by press fit. In some embodiments, a medical adhesive may be used to affix the magnets (or the casing) within the bores. In addition, an epoxy or similar medical adhesive may be used to fill the rest of the bore space once the magnet has been inserted. Such medical adhesives are known in the art.

**Kits**

An aspect of the present invention relates to a kit that can be used to prepare the hip prosthesis of the present invention.

In some embodiments, the kit may comprise a hip prosthesis and two or more magnets. The hip prosthesis may be a hip prosthesis as is known in the art, and the two or more magnets may be in accordance with the magnets described herein for the present invention.

In certain embodiments, the kit may further comprise a package insert. As used herein, "package insert" means a document that provides information on how to prepare the hip prosthesis of the present invention, for example, information based on the methods of manufacturing the hip prosthesis described herein. The package insert may further comprise safety information and other information required by a regulatory agency. A package insert can be a physical printed document in some embodiments.

In certain embodiments, the kit may further comprise one or more devices for preparing bores. Examples of such devices include, but are not limited to, a drill, a drill bit, and a combination thereof.

In certain embodiments, the kit may further comprise an adhesive for adhering the magnets after the magnets are inserted into the bores.

Alternatively, the kit may comprise a hip prosthesis that comprises bores in accordance with embodiments of the present invention, and two or more magnets. The bores may be positioned and oriented such that insertion of the magnets into the bores can result in the hip prosthesis that comprise magnets in accordance with embodiments of the present invention (*e.g.*, bores positioned at the central dome of the shell of the acetabular component, surrounding the central dome of the shell of the acetabular component, at end the surface in spherical head of the femoral component, *etc.*).

In other embodiments, the kit may comprise a hip prosthesis that comprises magnets in accordance with embodiments of the present invention, and a package insert as described herein.

**EXAMPLES**

**Example 1**

Modeling was used to simulate forces in a hip prosthesis comprising an acetabular component with a single cylindrical magnet at the central dome of the shell and a femoral component with a spherical magnet in the spherical head, in which the acetabular component and the femoral component were in a 0° hip position, in accordance with embodiments of the invention and as shown in **FIG. 5A.** The modeling was done using CST, a simulation technology that utilizes finite element analysis to calculate the magnetic forces and fields.

In the model, the acetabular shell of the acetabular component comprised an inner diameter of 50 mm. The cylindrical magnet of the acetabular shell comprised a diameter of 13 mm (~0.5 inches) and a height of 5 mm (~0.1875 inches), and was magnetized along its long axis. The spherical head of the femoral component comprised a diameter of 36 mm. The spherical magnet in the femoral head comprised a diameter of 16 mm (~0.625 inches), and was magnetized through the diameter. The surface-to-surface separation distance between the cylindrical magnet of the acetabular component and the spherical magnet of the femoral component was set at either 5 mm or 7 mm.

Both the cylindrical magnet of the acetabular component and the spherical magnet of the femoral component were assigned the material properties of N52 grade NdFeB alloy, including a remanence value of 1.48 Tesla (remanence is the magnetic field that remains in a material after an external magnetic field is removed). The environment was assumed to be air, which has the same magnetic permeability as non-ferrous alloys used in hip arthroplasty devices, polyethylene, ceramic, and water. The temperature was set to ambient temperature (25°C) as the magnetic permeability of the environment at ambient temperature is essentially unchanged from that at body temperature.

To determine the forces acting on a magnet in the femoral head from the magnet in the acetabular component, a proposed total hip system was first modeled as three-dimensional objects in SolidWorks (Dassault Systèmes, Waltham, MA) using magnet dimensions and idealized hip replacement system geometry. Once the geometry of the system was determined, the magnets were isolated and imported to CST Studio Suite (Dassault Systèmes, Vélizy-Villacoublay, France) for magnetic simulation calculations. CST is a finite element analysis software which uses simulation technology to accurately model, analyze, and quantify a wide range of electromagnetic phenomenon. This includes the ability to analyze complicated design geometries and, using a large materials database, solve for magnetic, electrical, thermal, and structural properties related to static permanent magnetic fields and electromagnetic fields.

The simulation software utilized an automatic tetrahedral meshing procedure to accurately approximate the geometry of the magnets used in the static simulations. After applying the refined surface meshes, the forces acting on each magnet were calculated and recorded for all simulations. The x, y, and z-components of the forces on each magnet were presented along with the net force between the femoral and acetabular magnet at 5mm and 7mm separation distances. The resulting magnetic flux density maps were generated. Magnetic flux density is the vector quantity that measures the strength (T, Tesla) of a magnetic field. The net force acting on the center of the femoral magnet and the directional components (x, y, and z, vectors) of the net force were then calculated using:

$$F = \frac{B^2 A}{2\,\mu_0}$$

where B is the flux density, A is the area of the surface exposed to the magnetic flux, and $\mu_0$ is the permeability of air ($4\pi \times 10^{-7}$ Tm/A). A positive directional component force represents an attractive force, and a negative directional component force represents a repulsive force. Due to symmetry of the models, all results are reported for the central coronal plane of the femoral head.

**Table 1** below provides the magnetic force acting on the spherical magnet in the femoral component at each separation distance, resulting from the attraction between the magnet of the acetabular component and the magnet of the femoral component. The net force was generated along the y-axis of the magnet, no x- or z- components of the attraction force. This is because the simulation is completely symmetric and the magnets were axially aligned, which is visualized by the uniform magnetic flux density vectors in the separation area between the femoral and acetabular component magnets as shown in the magnetic flux density map **(FIG. 6A)**

Table 1. Magnetic attraction forces between the spherical magnet in the femoral component and the magnet in the acetabular component for each separation distance. The results include the net attraction force and the attraction forces in the x-, y-, and z-directions (as shown in **FIG. 5A).**

| Separation Distance | Net Force | Directional Force | | |
|---|---|---|---|---|
| | | x | y | z |
| 5 mm | 13.1 N | 0.0 N | 13.1 N | 0.0 N |
| 7 mm | 8.5 N | 0.0 N | 8.5 N | 0.0 N |

As a comparison, forces were also simulated in a hip prosthesis shown in **FIG. 5B,** comprising an acetabular component with a single cylindrical magnet at the central dome of the shell and a femoral component with a single cylindrical magnet (identified in **FIG. 5B** with the reference numeral **40)** in the spherical head, in which the acetabular component and the femoral component were in a 0° hip position. The cylindrical magnet of the acetabular component comprised a diameter of 13 mm and a height of 5 mm, and the cylindrical magnet of the femoral component comprised a diameter of 16 mm and a height of 16 mm. The surface-to-surface separation distance between the cylindrical magnet of the acetabular component and the cylindrical magnet of the femoral component was set at either 5 mm or 7 mm. The magnets of both components were magnetized along their long axis and were assigned the material properties of N52 grade NdFeB alloy. The construction of the model and the conditions and performance of the simulation were as described above.

**Table 2** below provides the magnetic force acting on the cylindrical magnet in the spherical head of the femoral component at each separation distance, resulting from the attraction between the magnet of the femoral component and the magnet of the acetabular component.

**Table 2.** Magnetic attraction forces between the cylindrical magnet in the femoral component and the magnet in the acetabular component for each separation distance. The results include the net attraction force and the attraction forces in the x-, y-, and z-directions (as shown in **FIG. 5B**).

| Separation Distance | Net Force | Directional Force | | |
|---|---|---|---|---|
| | | x | y | z |
| 5 mm | 18.3 N | 0.0 N | 18.3 N | 0.0 N |
| 7 mm | 12.0 N | 0.0 N | 12.0 N | 0.0 N |

Based on this analysis, when the acetabular component comprises a single magnet at the central dome, the presence of a spherical magnet in the spherical head of the femoral component results in a lower attractive force as compared to presence of a cylindrical magnet in the spherical head of the femoral component. This is illustrated in the magnet flux density maps, in which the magnitude of the magnetic flux vectors between the femoral and acetabular magnets was less when the magnet of the femoral component was spherical **(FIG. 6A)** as compared to cylindrical **(FIG. 6B)**. The lower attractive force associated with the spherical magnet in the femoral component is likely due to the decreased volume of the spherical magnet as well as its curved surface, which increased the distance to the acetabular magnet.

**Example 2**

Modeling with CST was used to simulate forces in a hip prosthesis comprising an acetabular component with a single cylindrical magnet at the central dome in the shell and a femoral component with a spherical magnet in the spherical head, in which the acetabular component and the femoral component were in a 35° hip position, in accordance with embodiments of the invention and as shown in **FIG. 7A.**

In the model, the acetabular shell of the acetabular component comprised an inner diameter of 50 mm. The cylindrical magnet in the acetabular shell comprised a diameter of 13 mm and a height of 5 mm, and was magnetized along its long axis. The spherical head of the femoral component comprised a diameter of 36 mm. The spherical magnet in the femoral head comprised a diameter of 16 mm, and was magnetized through the diameter. The surface-to-surface separation distance between the cylindrical magnet of the acetabular component and the spherical magnet of the femoral component was set at either 5 mm or 7 mm. The magnets of both components were magnetized along their long axis and were assigned the material properties of N52 grade NdFeB alloy. The construction of the model and the conditions and performance of the simulation were as described in Example 1.

**Table 3** below provides the magnetic force acting on the spherical magnet in the femoral component at each separation distance, resulting from the attraction between the magnet of the femoral component and the magnet of the acetabular component.

**Table 3.** Magnetic attraction forces between the spherical magnet in the femoral component and the magnet in the acetabular component for each separation distance. The results include the net attraction force, the resultant force direction angle (θ, degrees) relative to the x-axis of the femoral head, and the attraction forces in the x-, y-, and z-directions (as shown in **FIG. 7A**).

| Separation Distance | Net Force | Resultant Force Direction | Directional Force | | |
|---|---|---|---|---|---|
| | | | x | y | z |
| 5 mm | 8.0 N | 29.7° | 7.0 N | 4.0 N | 0.0 N |
| 7 mm | 6.3 N | 40.0° | 4.9 N | 4.1 N | 0.0 N |

Further, forces were simulated in a hip prosthesis shown in **FIG. 7B,** comprising an acetabular component with a single cylindrical magnet at the central dome of the shell and a femoral component with a single cylindrical magnet (identified in **FIG. 7B** with the reference numeral **40)** in the spherical head, in which the acetabular component and the femoral component were in a 35° hip position. The cylindrical magnet of the acetabular component comprised a diameter of 13 mm and a height of 5 mm, and the cylindrical magnet of the femoral component comprised a diameter of 16 mm and a height of 16 mm. The surface-to-surface separation distance between the cylindrical magnet of the acetabular component and the cylindrical magnet of the femoral component was set at either 5 mm or 7 mm. The magnets of both components were magnetized along their long axis and were assigned the material properties of N52 grade NdFeB alloy. The construction of the model and the conditions and performance of the simulation were as described in Example 1.

**Table 4** below provides the magnetic force acting on the cylindrical magnet in the femoral component at each separation distance, resulting from the attraction between the magnet of the femoral component and the magnet of the acetabular component.

Table 4. Magnetic attraction forces between the cylindrical magnet in the femoral component and the magnet in the acetabular component for each separation distance. The results include the net attraction force, the resultant force direction angle (θ, degrees) relative to the x-axis of the femoral head, and the attraction forces in the x-, y-, and z-directions (as shown in **FIG. 7B).**

| Separation Distance | Net Force | Resultant Force Direction | Directional Force | | |
|---|---|---|---|---|---|
| | | | x | y | z |
| 5 mm | 7.1 N | -8.1 | 7.0 N | -1.0 N | 0.0 N |
| 7 mm | 5.9 N | 14.7° | 5.7 N | 1.5 N | 0.0 N |

At the 35° hip position, the net attractive forces between the magnets of the femoral head and the acetabular shell were reduced compared to the on-axis net forces in Example 1, for both the spherical magnet in the femoral head (compare **Table 3** and **Table 1)** and the cylindrical magnet in the femoral head (compare **Table 4** and **Table 2).** Also, the presence of a spherical magnet in the femoral component generated a stronger absolute attraction force towards the magnet in the acetabular component as compared to the cylindrical magnet in the femoral component. Further, both femoral magnet configurations had a lateral attractive x-component force of 7.0 N and a zero z-component force, but the cylindrical magnet had a small distractive force y-component force of -1.0 N, compared to an attractive y-component force of 4.0 N for the spherical magnet. This result implies a reaction force that represents a partial distractive force along the femoral head axis. While, in contrast, a cylindrical magnet in the femoral component generated forces away from the magnet in the acetabular component.

The magnetic flux density maps for simulations in which the femoral component comprise a spherical magnet **(FIG. 8A)** versus a cylindrical magnet **(FIG. 8B)** illustrate that the two femoral head magnet geometries generated different field maps, particularly where interaction of the opposing magnet fields occurs and in the areas adjacent to the upper right surface of the femoral magnet. For the spherical magnet, the rotational realignment and curved surface of the magnet allowed for a relatively smooth transition in the magnetic field between it and the acetabular shell magnet (see **FIG. 8A).** In contrast, for the cylindrical magnet, the greatest flux field occurred at the edge of the magnet closest to the acetabular magnet, and there was a sudden change in direction of the magnetic field lines near the top right corner of the femoral magnet (see **FIG. 8B).**

### Example 3

Modeling with CST was used to simulate forces in a hip prosthesis comprising an acetabular component having a shell with a single cylindrical magnet at the central dome and an array of four cylindrical magnets surrounding the single magnet, and a femoral component with a spherical magnet in the spherical head, in which the acetabular component and the femoral component were in a 0° hip position, in accordance with embodiments of the invention and as shown in **FIG. 9A.**

In the acetabular component of the model, the acetabular shell comprised an inner diameter of 50 mm. The single cylindrical magnet at the central dome of the shell comprised a diameter of 13 mm and a height of 5 mm, and the four magnets in the array, which were equidistant from the single magnet at the central dome and equidistant from each other, each comprised a diameter of 10 mm (~0.375 inches) and a height of 5 mm. These cylindrical magnets of the acetabular component were magnetized along their long axis.

In the femoral component, the spherical head comprised a diameter of 36 mm. The spherical magnet in the femoral head comprised a diameter of 16 mm, and was magnetized through the diameter.

The surface-to-surface separation distance between the cylindrical magnets of the acetabular component and the spherical magnet of the femoral component was set at either 5 mm or 7 mm. The magnets of both components were magnetized along their long axis and were assigned the material properties of N52 grade NdFeB alloy. The construction of the model and the conditions and performance of the simulation were as described in Example 1.

**Table 5** below provides the magnetic force acting on the spherical magnet in the femoral component at each separation distance, resulting from the attraction between the magnets of the acetabular component and the magnet of the femoral component. Comparing these results to the results in Example 1, at the 5 mm separation distance, the magnetic force acting on the spherical magnet in the spherical head is similar when the shell of the acetabular component comprised a single magnet at the central dome surrounded by the array and when the shell of the acetabular component comprised a single magnet at the central dome only. At the 7 mm separation distance, the magnetic force acting on the spherical magnet in the spherical head is greater when the shell of the acetabular component comprised a single magnet at the central dome surrounded by the array than when the shell of the acetabular component comprised a single magnet at the central dome only.

**Table 5.** Magnetic attraction forces between the spherical magnet in the femoral component and the magnets in the acetabular component for each separation distance. The results include the net attraction force and the attraction forces in the x-, y-, and z-directions (as shown in FIG. 9A).

| Separation Distance | Net Force | Directional Force | | |
|---|---|---|---|---|
| | | x | y | z |
| 5 mm | 12.6 N | 0.0 N | 12.6 N | 0.0 N |
| 7 mm | 10.5 N | 0.0 N | 10.5 N | 0.0 N |

In addition, forces were simulated in a hip prosthesis shown in **FIG. 9B,** comprising an acetabular component having a shell with a single cylindrical magnet at the central dome and an array of four cylindrical magnets surrounding the single magnet, and a femoral component with a single cylindrical magnet (identified in **FIG. 9B** with the reference numeral **40)** in the spherical head, in which the acetabular component and the femoral component were in a 0° hip position. In the shell of the acetabular component, the single cylindrical magnet at the central dome comprised a diameter of 13 mm and a height of 5 mm, and the four magnets in the array, which were equidistant from the single magnet at the central dome and equidistant from each other, each comprised a diameter of 10 mm and a height of 5 mm. In the spherical head of the femoral component, the cylindrical magnet comprised a diameter of 16 mm and a height of 16 mm. The surface-to-surface separation distance between the cylindrical magnets of the acetabular component and the cylindrical magnet of the femoral component was set at either 5 mm or 7 mm. The magnets of both components were magnetized along their long axis and were assigned the material properties of N52 grade NdFeB alloy. The construction of the model and the conditions and performance of the simulation were as described in Example 1.

**Table 6** below provides the magnetic force acting on the cylindrical magnet in the femoral component at each separation distance, resulting from the attraction between the magnet of the femoral component and the magnets of the acetabular component.

**Table 6.** Magnetic attraction forces between the cylindrical magnet in the femoral component and the magnets in the acetabular component for each separation distance. The results include the net attraction force and the attraction forces in the x-, y-, and z-directions (as shown in **FIG. 9B).**

| Separation Distance | Net Force | Directional Force | | |
|---|---|---|---|---|
| | | x | y | z |
| 5 mm | 12.5 N | 0.0 N | 12.5 N | 0.0 N |
| 7 mm | 10.4 N | 0.0 N | 10.4 N | 0.0 N |

These results show that, when the shell of the acetabular component comprises an array of magnets surrounding a single magnet at the central dome, the attractive force on the magnet in the spherical head of the femoral component is similar despite whether the magnet in the spherical head comprises a spherical shape or comprises a cylindrical shape.

For the spherical magnet in the femoral component, the magnetic field flux density map illustrates a smooth transition of the magnetic field around the femoral head magnet in response to the geometry of the magnet due to the array and an absence of unstable regions of the magnetic field directly adjacent to the femoral head magnet (see **FIG. 10A**). For the cylindrical magnet in the femoral component, the center magnet in the acetabular shell influenced the magnetic field and the resulting attraction force to a greater extent, despite the dampening of the field by the surrounding off-axis magnetic array (see **FIG. 10B**). The magnetic attraction force was consistent between cylindrical and spherical femoral head magnets despite the sphere having a smaller volume and larger distance between its surface and the array magnets.

**Example 4**

Modeling with CST was used to simulate forces in a hip prosthesis comprising an acetabular component having a shell with a single cylindrical magnet at the central dome and an array of four cylindrical magnets surrounding the single magnet, and a femoral component with a spherical magnet in the spherical head, in which the acetabular component and the femoral component were in a 35° hip position,, in accordance with embodiments of the invention and as shown in **FIG. 11A.**

In the acetabular component of the model, the acetabular shell comprised an inner diameter of 50 mm. The single cylindrical magnet at the central dome of the shell comprised a diameter of 13 mm and a height of 5 mm, and the four magnets in the array, which were equidistant from the single magnet at the central dome and equidistant from each other, each comprised a diameter of 10 mm and a height of 5 mm. These cylindrical magnets of the acetabular component were magnetized along their long axis.

In the femoral component, the spherical head comprised a diameter of 36 mm. The spherical magnet in the femoral head comprised a diameter of 16 mm, and was magnetized through the diameter.

The surface-to-surface separation distance between the cylindrical magnets of the acetabular component and the spherical magnet of the femoral component was set at either 5 mm or 7 mm. The magnets of both components were magnetized along their long axis and were assigned the material properties of N52 grade NdFeB alloy. The construction of the model and the conditions and performance of the simulation were as described in Example 1.

**Table 7** below provides the magnetic force acting on the spherical magnet in the femoral component at each separation distance, resulting from the attraction between the magnet of the femoral component and the magnets of the acetabular component. Comparing these results to the results in Example 2, at both separation distances, the magnetic force acting on the spherical magnet in the femoral component are greater when the shell of the acetabular component comprised a single magnet at the central dome of the shell and an array of magnets surrounding the single magnet, than when the shell comprised a single magnet at the central dome only.

**Table 7.** Magnetic attraction forces between the spherical magnet in the femoral component and the magnets in the acetabular component for each separation distance. The results include the net attraction force, the resultant force direction angle (θ, degrees) relative to the x-axis of the femoral head, and the attraction forces in the x-, y-, and z-directions (as shown in FIG. 11A).

| Separation Distance | Net Force | Resultant Force Direction | Directional Force | | |
|---|---|---|---|---|---|
| | | | x | y | z |
| 5 mm | 10.8 N | 50.1° | 6.8 N | 8.3 N | 0.0 N |
| 7 mm | 9.7 N | 48.8° | 6.4 N | 7.3 N | 0.0 N |

Moreover, forces were simulated in a hip prosthesis shown in **FIG. 11B,** comprising an acetabular component having a shell with a single cylindrical magnet at the central dome and an array of four cylindrical magnets surrounding the single magnet, and a femoral component with a single cylindrical magnet (identified in **FIG. 11B** with the reference numeral **40)** in the spherical head, in which the acetabular component and the femoral component were in a 35° hip position. In the shell of the acetabular component, the single cylindrical magnet at the central dome comprised a diameter of 13 mm and a height of 5 mm, and the four magnets in the array, which were equidistant from the single magnet at the central dome and equidistant from each other, each comprised a diameter of 10 mm and a height of 5 mm. In the spherical head of the femoral component, the cylindrical magnet comprised a diameter of 16 mm and a height of 16 mm. The surface-to-surface separation distance between the cylindrical magnets of the acetabular component and the cylindrical magnet of the femoral component was set at either 5 mm or 7 mm. The magnets of both components were magnetized along their long axis and were assigned the material properties of N52 grade NdFeB alloy. The construction of the model and the conditions and performance of the simulation were as described in Example 1.

**Table 8** below provides the magnetic force acting on the cylindrical magnet in the femoral component at each separation distance, resulting from the attraction between the magnet of the femoral component and the magnets of the acetabular component.

**Table 8.** Magnetic attraction forces between the cylindrical magnet in the femoral component and the magnets in the acetabular component for each separation distance. The results include the net attraction force, the resultant force direction angle ($\theta$, degrees) relative to the x-axis of the femoral head, and the attraction forces in the x-, y-, and z-directions (as shown in **FIG. 11B**).

| Separation Distance | Net Force | Resultant Force Direction | Directional Force | | |
|---|---|---|---|---|---|
| | | | x | y | z |
| 5 mm | 11.0 N | 32.4° | 9.3 N | 5.9 N | 0.0 N |
| 7 mm | 10.3 N | 27.1° | 9.2 N | 4.7 N | 0.0 N |

These results show that, while the absolute acting force on the femoral magnet was similar when the femoral magnet was spherical as compared to cylindrical, the components and angle of those forces were not. When the femoral magnet is spherical, the force vector was directed primarily in line with the rotation of the acetabular shell (50.1° and 48.8° at 5mm and 7mm separation distance), with only attractive forces in the x- and y-axis present. The net force vector maintained the attractive force between the femoral head and the acetabular shell in alignment central to the rotation of the head. Discontinuities in the magnetic flux field were minimized and the attractive force reaction at the femoral head increased compared to the cylindrical magnet (see **FIG. 11A).**

In comparison, as shown in the magnetic flux density map for when the femoral component comprised a cylindrical magnet (see **FIG 11B**), there is a greater flux density in the area above the top right of the cylindrical femoral head magnet than the top left. The net force at the cylindrical femoral head magnet is a combination of the force directed toward the acetabular central dome magnet in the x-direction (9.3 N) and the attraction to the magnet in the array directly above the femoral head cylindrical magnet in the y-direction (5.9 N). At 7 mm separation, the net attractive force on the femoral head decreased to 10.3 N, dampening effects on the y-component force (4.7 N) and directing it along the x-axis (27.1°).

Thus, this configuration demonstrates the benefit of having a spherical magnet rather than a cylindrical magnet in the femoral component. A strong x-component of the force acting on the femoral head magnet will generate resistance when walking, while a strong y-component will ensure the femoral head remains in contact with the acetabular component. The rotating spherical magnet in the femoral head generates a smaller x-component and a larger y-component while maintaining the same net force as the cylindrical magnet.

**Example 5**

Validation of the simulation results of Examples 1-4 was conducted via mechanical testing. Three-dimensional printed models of an acetabular shell and a femoral stem taper designed to house the femoral head magnet were produced allowing for placement of NdFeB magnets of the dimensions and type used in the computer simulations. The components were mounted to a servo-hydraulic test system (Instron 8874, Norwood, MA) and tested in displacement control at a rate of 10 mm/min. A single axis load cell was attached to the femoral head magnet fixture to record the long axis (y) reaction force in response to the increasing separation distance between the femoral head magnet and the acetabular component magnet or array of magnets. The reaction load was recorded at a minimum separation distance of 2 mm to a maximum separation of 30 mm. The y-component reaction force at the modelled separation distances of 5 mm and 7 mm was directly compared to the mechanical testing results in the y-axis direction. All tests were repeated twice and mean load calculated at 5 mm and 7 mm separation distances.

The mechanical test results were reasonably predicted by simulations. A summary of the measured reaction force along the femoral head axis at 5 mm and 7 mm separation distance is shown in **Table 9.** Similar to the simulation results, at 5 mm separation and 0° hip position, the configuration in which there was a single magnet in both the acetabular component and the femoral component generated the greatest on-axis attractive force measured: 12.1 N for a spherical magnet in the femoral component, and 15.7 N for a cylindrical magnet in the femoral component. At the 7 mm separation distance, the load at the femoral head magnet decreased for both the spherical and cylindrical magnets.

At the 35° hip position and the configuration in which there was a single magnet in both the acetabular component and the femoral component, there was a smaller decrease in y-axis reaction force when a spherical magnet was in the femoral component than when a cylindrical magnet was in the femoral component (5.5 N and 4.9 N at 5 mm and 7 mm separation distance for the spherical magnet, versus 0.9 N and 1.8 N, respectively, for the cylindrical magnet). The simulation predicted the reaction force at the spherical magnet in this configuration, but the direction of the y-axis reaction load measured at the femoral head with the cylindrical magnet had a positive y-component rather than a negative direction.

At the 0° hip position and the configuration in which there was a single magnet plus an array of magnets in the acetabular component, the y-axis attractive force was decreased for both femoral head magnets compared to pairing with the single central magnet in the acetabular component, as was shown in the simulation results. The spherical head magnet had nearly the same y-axis force as the cylindrical magnet when paired with the single magnet+ array in the acetabular component (10.3 N and 9.2 N for the cylindrical magnet vs. 9.9 N and 8.2 N for the spherical magnet at 5 mm and 7 mm, respectively). At the 35° hip position, a similar trend occurred; however, the overall y-axis force was greater for the spherical femoral head magnet compared to the cylindrical magnet (6.6 N and 6.0 N for the spherical at 5mm and 7mm separation distance, respectively, vs. 2.7 N and 2.4N for the cylindrical magnet).

Thus, the overall forces measured by mechanical testing were in reasonable agreement with those predicted by the simulations.

**Table** 9. Measured reaction force (N) along the femoral head axis (y) at different magnet separation distances between magnet(s) of acetabular component and spherical or cylindrical magnet of the femoral component, as determined using mechanical testing.

| Configuration | | | Separation Distance | |
|---|---|---|---|---|
| **Acetabular Magnet** | **Femoral Magnet** | **Hip Position** | **5 mm** | **7 mm** |
| Single, cylindrical | Single, spherical | 0 | 12.1 N | 7.9 N |
| Single, cylindrical | Single, cylindrical | 0 | 15.7 N | 10.4 N |
| Single, cylindrical | Single, spherical | 35 | 5.5 N | 4.9 N |
| Single, cylindrical | Single, cylindrical | 35 | 0.9 N | 1.8 N |
| Array, cylindrical | Single, spherical | 0 | 9.9 N | 8.2 N |
| Array, cylindrical | Single, cylindrical | 0 | 10.3 N | 9.2 N |
| Array, cylindrical | Single, spherical | 35 | 6.6 N | 6.0 N |
| Array, cylindrical | Single, cylindrical | 35 | 2.7 N | 2.4 N |

The invention is not to be limited in scope by the specific embodiments described herein. Indeed, various modifications of the invention in addition to those described will become apparent to those skilled in the art from the foregoing description and accompanying Figures. Such modifications are intended to fall within the scope of the appended claims.

All references cited herein are incorporated herein by reference in their entirety and for all purposes to the same extent as if each individual publication or patent or patent application was specifically and individually indicated to be incorporated by reference in its entirety for all purposes.

# EP 4 710 897 A1

## REFERENCES

[1] Gillinov SM, et al., Incidence, timing, and predictors of hip dislocation after primary total hip arthroplasty for osteoarthritis, Journal of the American Academy of Orthopaedic Surgeons, 2022, 30(21):1047-1053. d

[2] Ullmark G, The unstable total hip arthroplasty, EFORT Open Reviews, 2016, 1: 83-88.

[3] Dargel J, et al., Dislocation following total hip replacement, Deutsches Arzteblatt International, 2014, 111(51-52): 884-90.

[4] Faldini C, et al., How to prevent dislocation after revision total hip arthroplasty: a systematic review of the risk factors and a focus on treatment options, Journal of Orthopaedics and Traumatology, 2018, 19(1):17.

[5] Zahar A, et al., Dislocation after total hip arthroplasty, Current Reviews in Musculoskeletal Medicine, 2013, 6(4): 350-356.

[6] Guyen O, Constrained liners, dual mobility or large diameter heads to avoid dislocation in THA, EFORT Open Reviews, 2017, 1(5): 197-204.

[7] Noble PC, et al., Constrained cups appear incapable of meeting the demands of revision THA, Clinical Orthopaedics and Related Research, 2012, 470(7): 1907-1916.

[8] Jones SA, Constrained acetabular liners, The Journal of Arthroplasty, 2018, 33(5): 1331-1336.

[9] Yang S, et al., A review of the clinical and engineering performance of dual-mobility cups for total hip arthroplasty, American Journal of Translational Research, 2021, 13(8): 9383-9394.

## EMBODIMENTS

Various preferred features and embodiments of the present invention will now be described with reference to the following numbered paragraphs (paras).

1. A hip prosthesis, comprising

(a) an acetabular component comprising a shell having an approximate shape of a hollowed spherical cap that comprises a central dome, a periphery, and an intermediate wall therebetween, wherein the shell comprises a concave inner surface, a convex outer surface, a thickness therebetween, and one or more magnets; and
(b) a femoral component comprising

(i) a stem comprising a proximal end and a distal end,
(ii) a neck comprising a tapered end and a base end, wherein the base end of the neck is joined to the proximal end of the stem, and the neck extends at an angle from the stem,
(iii) a spherical head that is affixed to the tapered end of the neck, wherein the spherical head comprises a spherical magnet, and wherein the spherical magnet is rotatable within the spherical head;

wherein the spherical head comprises a tapered volume, an outside surface, and a thickness between the tapered volume and the outside surface, and wherein the tapered volume is configured to receive the tapered end of the neck;
wherein the acetabular component is configured to receive all or a portion of the spherical head of the femoral component; and
wherein the one or more magnets of the shell of the acetabular component and the spherical magnet of the spherical head of the femoral component are oriented to generate an attractive force therebetween.

2. The hip prosthesis of para 1, wherein the convex inner surface of the shell is configured to articulate with the outer surface of the spherical head.

3. The hip prosthesis of para 1 or 2, wherein the acetabular component further comprises a liner that comprises a concave inner surface, a convex outer surface, and a thickness therebetween, wherein the concave inner surface of the shell is configured to receive all or a portion of the convex outer surface of the liner.

4. The hip prosthesis of para 3, wherein the convex inner surface of the liner is configured to articulate with the outer surface of the spherical head.

5. The hip prosthesis of any one of paras 1-4, wherein the one or more magnets of the shell comprise a cylindrical, prism, conal, or pyramidal shape.

6. The hip prosthesis of para 5, wherein the one or more magnets of the shell comprise a cylindrical shape.

7. The hip prosthesis of para 6, wherein the one or more magnets of the shell comprise a diameter of about 2 mm to about 20 mm, and a length of about 1 mm to about 15 mm.

8. The hip prosthesis of para 7, wherein the one or more magnets of the shell comprise a diameter of about 4 mm to about 15 mm, and a length of about 3 mm to about 10 mm.

9. The hip prosthesis of any one of paras 4-8, wherein the one or more magnets of the shell are on the concave surface of the shell.

10. The hip prosthesis of any one of paras 1-8, wherein the one or more magnets of the shell are in the thickness of the shell.

11. The hip prosthesis of any one of paras 1-10, wherein the one or more magnets of the shell are at or adjacent to the central dome.

12. The hip prosthesis of para 11, wherein the one or more magnets of the shell comprise a single magnet at or adjacent to the central dome.

13. The hip prosthesis of para 12, wherein the one or more magnets of the shell further comprise an array of magnets surrounding the single magnet.

14. The hip prosthesis of para 13, wherein each magnet in the array of magnets is equidistant from the single magnet.

15. The hip prosthesis of para 13 or 14, wherein each magnet in the array of magnets is equidistant from each other.

16. The hip prosthesis of any one of paras 12-15, wherein the single magnet at the central dome is oriented such that its long axis is perpendicular to the tangent line of the curvature at the central dome.

17. The hip prosthesis of any one of paras 13-16, wherein the magnets in the array are oriented such that their long axes are parallel to the long axis of the single magnet.

18. The hip prosthesis of any one of paras 13-16, wherein the magnets in the array are oriented such that their long axes are angled to the long axis of the single magnet.

19. The hip prosthesis of para 18, wherein the angle between the long axis of the magnets in the array and the long axis of the single magnet is about 10° to about 80°.

20. The hip prosthesis of para 19, wherein the angle between the long axis of the magnets in the array and the long axis of the single magnet is about 30° to about 60°.

21. The hip prosthesis of para 11, wherein the one or more magnets of the shell comprise an array of magnets adjacent to the central dome.

22. The hip prosthesis of para 21, wherein each magnet in the array of magnets is equidistant from the central dome.

23. The hip prosthesis of para 21 or 22, wherein each magnet in the array of magnets is equidistant from each other.

24. The hip prosthesis of any one of paras 21-23, wherein the magnets in the array are oriented such that their long axes are perpendicular to the tangent line of the curvature at the central dome.

25. The hip prosthesis of any one of paras 21-23, wherein the magnets in the array are oriented such that their long axes are angled to a line that is perpendicular to the tangent line of the curvature at the central dome.

26. The hip prosthesis of para 25, wherein the angle between the long axis of the magnets in the array and the line that is perpendicular to the tangent line of the curvature at the central dome is about 10° to about 80°.

27. The hip prosthesis of para 26, wherein the angle between the long axis of the magnets in the array and the line that is perpendicular to the tangent line of the curvature at the central dome is about 30° to about 60°.

28. The hip prosthesis of any one of paras 13-27, wherein the array of magnets comprises between 2 and 16 magnets.

29. The hip prosthesis of para 28, wherein the array of magnets comprises between 2 and 8 magnets.

30. The hip prosthesis of para 29, wherein the array of magnets comprises between 2 and 6 magnets.

31. The hip prosthesis of para 30, wherein the array of magnets comprises 4 magnets.

32. The hip prosthesis of any one of paras 1-31, wherein the one or more magnets of the shell comprise a magnetic material of a rare-earth magnet.

33. The hip prosthesis of any one of paras 1-32, wherein the one or more magnets of the shell comprise a magnetic material of an alloy of neodymium, iron, and boron.

34. The hip prosthesis of any one of paras 1-33, wherein the one or more magnets of the shell are magnetized along the long axis of the magnets.

35. The hip prosthesis of any one of paras 1-34, wherein the one or more magnets of the shell comprise a casing that encloses the magnetic material.

36. The hip prosthesis of any one of paras 1-35, wherein the tapered volume of the spherical head extends inward from the outside surface of the spherical head.

37. The hip prosthesis of any one of paras 1-36, wherein, the tapered volume of the spherical head is defined by an end surface that is the innermost surface of the tapered volume, and walls that extend from the outside surface to the end surface.

38. The hip prosthesis of any one of paras 1-37, wherein the cross-sectional area of the tapered volume of the spherical head decreases towards the end surface from the outside surface of the spherical head.

39. The hip prosthesis of para 37 or 38, wherein the spherical magnet of the spherical head is at or adjacent to the end surface of the tapered volume.

40. The hip prosthesis of any one of paras 1-39, wherein the spherical head further comprises a casing surrounding the spherical magnet, and wherein the spherical magnet is rotatable within the casing.

41. The hip prosthesis of any one of paras 1-40, wherein the spherical magnet of the spherical head comprise a diameter of about 1 mm to about 30 mm.

42. The hip prosthesis of para 41, wherein the spherical magnet of the spherical head comprises a diameter of about 5 mm to about 20 mm.

43. The hip prosthesis of any one of paras 1-42, wherein the spherical magnet of the spherical head comprises a magnetic material of a rare-earth magnet.

44. The hip prosthesis of any one of paras 1-43, wherein the spherical magnet of the spherical head comprises a magnetic material of an alloy of neodymium, iron, and boron.

45. The hip prosthesis of any one of paras 1-44, wherein the spherical magnet of the spherical head is magnetized through its diameter.

46. The hip prosthesis of any one of paras 1-45 for use in treating a subject in need of a total hip replacement.

47. The hip prosthesis of any one of paras 1-45 for use in stabilizing a total hip replacement prosthesis in a subject.

48. The hip prosthesis of any one of paras 1-45 for use in reducing incidence of total hip replacement dislocation in a subject.

49. The hip prosthesis of any one of paras 1-45 for use in reducing risk of total hip replacement dislocation in a subject.

50. The hip prosthesis of para 48 or 49, wherein the total hip replacement dislocation is due to impingement.

51. The hip prosthesis of any one of paras 1-45 for use in reducing risk of impingement associated with total hip replacement in a subject.

52. The hip prosthesis of any one of paras 1-45 for use in reducing risk of total hip replacement subluxation in a subject.

53. The hip prosthesis of any one of paras 1-45 for use in reducing osteolysis associated with total hip replacement in a subject.

54. A method of treating a subject in need of a total hip replacement, the method comprising implanting the hip prosthesis of any one of paras 1-45 in the subject.

55. A method of stabilizing a total hip replacement prosthesis in a subject, the method comprising implanting the hip prosthesis of any one of paras 1-45 in the subject.

56. The method of para 54 or 55, wherein the hip prosthesis stabilizes the hip joint during healing after implantation.

57. A method of reducing incidence of total hip replacement dislocation in a subject, the method comprising implanting the hip prosthesis of any one of paras 1-45 in the subject.

58. A method of reducing risk of total hip replacement dislocation in a subject, the method comprising implanting the hip prosthesis of any one of paras 1-45 in the subject.

59. The method of para 57 or 58, wherein the total hip replacement dislocation is due to impingement.

60. A method of reducing risk of impingement associated with total hip replacement in a subject, the method comprising implanting the hip prosthesis of any one of paras 1-45 in the subject.

61. A method of reducing risk of total hip replacement subluxation in a subject, the method comprising implanting the hip prosthesis of any one of paras 1-45 in the subject.

62. A method of reducing osteolysis associated with total hip replacement in a subject, the method comprising implanting the hip prosthesis of any one of paras 1-45 in the subject.

63. Use of the hip prosthesis of any one of paras 1-45 for treating a subject in need of a hip, the use comprising implanting the hip prosthesis in the subject.

64. Use of the hip prosthesis of any one of paras 1-45 for stabilizing a total hip replacement prosthesis in a subject, the use comprising implanting the hip prosthesis in the subject.

65. The use of para 63 or 64, wherein the hip prosthesis stabilizes the hip joint during healing after implantation.

66. Use of the hip prosthesis of any one of paras 1-45 for reducing incidence of total hip replacement dislocation in a subject, the use comprising implanting the hip prosthesis in the subject.

67. Use of the hip prosthesis of any one of paras 1-45 for reducing risk of total hip replacement dislocation in a subject, the use comprising implanting the hip prosthesis in the subject.

68. The use of para 66 or 67, wherein the total hip replacement dislocation is due to impingement.

69. Use of the hip prosthesis of any one of paras 1-45 for reducing risk of impingement associated with total hip replacement in a subject.

70. Use of the hip prosthesis of any one of paras 1-45 for reducing risk of total hip replacement subluxation in a subject, the use comprising implanting the hip prosthesis in the subject.

71. Use of the hip prosthesis of any one of paras 1-45 for reducing osteolysis associated with total hip replacement in a subject, the use comprising implanting the hip prosthesis in the subject.

72. A femoral component of a hip prosthesis, comprising

(a) a stem comprising a proximal end and a distal end,
(b) a neck comprising a tapered end and a base end, wherein the base end of the neck is joined to the proximal end of the stem, and the neck extends at an angle from the stem,
(c) a spherical head that is affixed to the tapered end of the neck, wherein the spherical head comprises a spherical magnet, and wherein the spherical magnet is rotatable within the spherical head;

wherein the spherical head comprises a tapered volume, an outside surface, and a thickness between the tapered volume and the outside surface, and wherein the tapered volume is configured to receive the tapered end of the neck.

## Claims

1. A hip prosthesis, comprising

(a) an acetabular component comprising a shell having an approximate shape of a hollowed spherical cap that comprises a central dome, a periphery, and an intermediate wall therebetween, wherein the shell comprises a concave inner surface, a convex outer surface, a thickness therebetween, and one or more magnets; and
(b) a femoral component comprising

(i) a stem comprising a proximal end and a distal end,
(ii) a neck comprising a tapered end and a base end, wherein the base end of the neck is joined to the proximal end of the stem, and the neck extends at an angle from the stem,
(iii) a spherical head that is affixed to the tapered end of the neck, wherein the spherical head comprises a spherical magnet, and wherein the spherical magnet is rotatable within the spherical head;

wherein the spherical head comprises a tapered volume, an outside surface, and a thickness between the tapered volume and the outside surface, and wherein the tapered volume is configured to receive the tapered end of the neck;
wherein the acetabular component is configured to receive all or a portion of the spherical head of the femoral component; and
wherein the one or more magnets of the shell of the acetabular component and the spherical magnet of the spherical head of the femoral component are oriented to generate an attractive force therebetween.

2. The hip prosthesis of claim 1, wherein the convex inner surface of the shell is configured to articulate with the outer surface of the spherical head.

3. The hip prosthesis of claim 1 or 2, wherein the acetabular component further comprises a liner that comprises a concave inner surface, a convex outer surface, and a thickness therebetween, wherein the concave inner surface of the shell is configured to receive all or a portion of the convex outer surface of the liner.

4. The hip prosthesis of claim 3, wherein the convex inner surface of the liner is configured to articulate with the outer surface of the spherical head.

5. The hip prosthesis of any one of claims 1-4, wherein the one or more magnets of the shell comprise a cylindrical shape.

6. The hip prosthesis of any one of claims 1-5, wherein the one or more magnets of the shell comprise a single magnet at or adjacent to the central dome.

7. The hip prosthesis of claim 6, wherein the one or more magnets of the shell further comprise an array of magnets surrounding the single magnet.

8. The hip prosthesis of claim 7, wherein each magnet in the array of magnets is equidistant from the single magnet.

9. The hip prosthesis of any one of claims 6-8, wherein the single magnet at the central dome is oriented such that its long axis is perpendicular to the tangent line of the curvature at the central dome.

10. The hip prosthesis of any one of claims 7-9, wherein the array of magnets comprises between 2 and 8 magnets.

11. The hip prosthesis of any one of claims 1-10, wherein the one or more magnets of the shell are magnetized along the long axis of the magnets.

12. The hip prosthesis of any one of claims 1-11, wherein the spherical head further comprises a casing surrounding the spherical magnet, and wherein the spherical magnet is rotatable within the casing.

13. The hip prosthesis of any one of claims 1-12, wherein the spherical magnet of the spherical head is magnetized through its diameter.

14. The hip prosthesis of any one of claims 1-13, for use in:

>(i) treating a subject in need of a total hip replacement;
>(ii) stabilizing a total hip replacement prosthesis in a subject;
>(iii) reducing incidence of total hip replacement dislocation in a subject;
>(iv) reducing risk of total hip replacement dislocation in a subject;
>(v) reducing risk of impingement associated with total hip replacement in a subject;
>(vi) reducing risk of total hip replacement subluxation in a subject; or
>(vii) reducing osteolysis associated with total hip replacement in a subject.

15. A femoral component of a hip prosthesis, comprising

>(a) a stem comprising a proximal end and a distal end,
>(b) a neck comprising a tapered end and a base end, wherein the base end of the neck is joined to the proximal end of the stem, and the neck extends at an angle from the stem,
>(c) a spherical head that is affixed to the tapered end of the neck, wherein the spherical head comprises a spherical magnet, and wherein the spherical magnet is rotatable within the spherical head;

wherein the spherical head comprises a tapered volume, an outside surface, and a thickness between the tapered volume and the outside surface, and wherein the tapered volume is configured to receive the tapered end of the neck.

**FIG. 1**

A

21

28

25

23

22

26

27

24

FIG. 2

**B**

30  35

34

28

33

31

32

29

FIG. 2 (cont.)

**A**

2

34

13'

36

28

35

1

**B**

2

28

13'

34

35

1

**FIG. 3**

A

35°

FIG. 4

A

2    1

13'

36

34

35

28

B

2    1

13'

36

40

28

FIG. 5

A

B

FIG. 6

FIG. 7

A

B

**FIG. 8**

FIG. 9

A

B

**FIG. 10**

**FIG. 11**

A

B

FIG. 12

Europäisches
Patentamt

European
Patent Office

Office européen
des brevets

# EUROPEAN SEARCH REPORT

**Application Number**

EP 25 19 2881

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2008/057565 A2 (HOWMEDICA OSTEONICS CORP [US]; ZHANG ZONGTAO [US] ET AL.) 15 May 2008 (2008-05-15) * paragraph [0034] - paragraph [0037] * * paragraph [0060] - paragraph [0061] * * figure 7 * | 1-15 | INV. A61F2/32 A61F2/36 |
| A | US 2018/014838 A1 (NING AUTUMN [US]) 18 January 2018 (2018-01-18) * paragraph [0041] * * figure 4 * | 1,15 | |
| A | US 4 024 588 A (JANSSEN RAINER ET AL) 24 May 1977 (1977-05-24) * column 4, line 63 - column 5, line 15 * * figure 4 * | 1,15 | |
| A | EP 3 977 964 A1 (FELLOWSHIP OF ORTHOPAEDIC RES INC [US]) 6 April 2022 (2022-04-06) * paragraph [0147] * * figures * | 1,15 | |

**TECHNICAL FIELDS SEARCHED (IPC)**

A61F

The present search report has been drawn up for all claims

| Place of search | Date of completion of the search | Examiner |
|---|---|---|
| Munich | 14 October 2025 | Storer, John |

EPO FORM 1503 03.82 (P04C01)

## ANNEX TO THE EUROPEAN SEARCH REPORT
## ON EUROPEAN PATENT APPLICATION NO.

EP 25 19 2881

This annex lists the patent family members relating to the patent documents cited in the above-mentioned European search report.
The members are as contained in the European Patent Office EDP file on
The European Patent Office is in no way liable for these particulars which are merely given for the purpose of information.

14-10-2025

| Patent document cited in search report | | Publication date | Patent family member(s) | | Publication date |
|---|---|---|---|---|---|
| WO 2008057565 | A2 | 15-05-2008 | NONE | | |
| US 2018014838 | A1 | 18-01-2018 | AU | 2017290158 A1 | 14-03-2019 |
| | | | US | 2018014838 A1 | 18-01-2018 |
| | | | WO | 2018005792 A1 | 04-01-2018 |
| US 4024588 | A | 24-05-1977 | NONE | | |
| EP 3977964 | A1 | 06-04-2022 | AU | 2021232776 A1 | 21-04-2022 |
| | | | CA | 3132490 A1 | 01-04-2022 |
| | | | EP | 3977964 A1 | 06-04-2022 |
| | | | US | 2022104947 A1 | 07-04-2022 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 02600725
- US 63694572

**Non-patent literature cited in the description**

- **GILLINOV SM et al.** Incidence, timing, and predictors of hip dislocation after primary total hip arthroplasty for osteoarthritis. *Journal of the American Academy of Orthopaedic Surgeons*, 2022, vol. 30 (21), 1047-1053
- **ULLMARK G**. The unstable total hip arthroplasty. *EFORT Open Reviews*, 2016, vol. 1, 83-88
- **DARGEL J et al.** Dislocation following total hip replacement. *Deutsches Arzteblatt International*, 2014, vol. 111 (51-52), 884-90
- **FALDINI C et al.** How to prevent dislocation after revision total hip arthroplasty: a systematic review of the risk factors and a focus on treatment options. *Journal of Orthopaedics and Traumatology*, 2018, vol. 19 (1), 17
- **ZAHAR A et al.** Dislocation after total hip arthroplasty. *Current Reviews in Musculoskeletal Medicine*, 2013, vol. 6 (4), 350-356
- **GUYEN O**. Constrained liners, dual mobility or large diameter heads to avoid dislocation in THA. *EFORT Open Reviews*, 2017, vol. 1 (5), 197-204
- **NOBLE PC et al.** Constrained cups appear incapable of meeting the demands of revision THA. *Clinical Orthopaedics and Related Research*, 2012, vol. 470 (7), 1907-1916
- **JONES SA**. Constrained acetabular liners. *The Journal of Arthroplasty*, 2018, vol. 33 (5), 1331-1336
- **YANG S et al.** A review of the clinical and engineering performance of dual-mobility cups for total hip arthroplasty. *American Journal of Translational Research*, 2021, vol. 13 (8), 9383-9394